# EUROPEAN PATENT SPECIFICATION

(11) **EP 2 524 379 B1**
(45) Date of publication and mention of the grant of the patent: **03.09.2014**
(21) Application number: 11703518.8
(22) Date of filing: 14.01.2011
(51) Int. Cl.: G21F 3/00, G21F 3/02, A61B 6/10, A61B 6/00

(54) **IMPROVED SYSTEM AND METHOD FOR PROVIDING A SUSPENDED PERSONAL RADIATION PROTECTION SYSTEM**
VERBESSERTES SYSTEM UND VERFAHREN ZUR BEREITSTELLUNG EINES HÄNGENDEN PERSÖNLICHEN STRAHLUNGSSCHUTZSYSTEMS
SYSTÈME AMÉLIORÉ ET PROCÉDÉ FOURNISSANT UN SYSTÈME SUSPENDU DE PROTECTION PERSONNELLE CONTRE LE RAYONNEMENT

(30) Priority: 15.01.2010 US 688353; 22.01.2010 US 692199
(43) Date of publication of application: 21.11.2012
(73) Proprietor: Interventco, LLC, Dallas, TX 75225 (US)
(72) Inventor: REES, Chet, R., Dallas, TX 75225 (US)
(74) Representative: Gosnall, Toby
(86) International application number: PCT/US2011/021330
(87) International publication number: WO 2011/088341

(56) References cited:
- US-A- 5 274 851
- US-A1- 2009 184 269
- US-A1- 2009 256 044

## Description

### CROSS-REFERENCE TO RELATED APPLICATIONS

This application claims the benefit of and priority to co-pending U.S. patent application Serial No. 12/692,199, filed on January 22, 2010, which claims benefit of and priority to U.S. patent application Serial No. 12/688,353, filed on January 15, 2010, which claims the benefit of and priority to co-pending U.S. patent application Serial No. 12/099,077, filed on April 7, 2008, which claims the benefit of and priority to U.S. provisional patent application Serial No. 61/022,174, filed on January 18, 2008. This application also claims the benefit of and priority to co-pending U.S. patent application Serial No. 12/557,703, filed on September 11, 2009, which claims the benefit of and priority to U.S. patent application Serial No. 11/611,627, filed on December 15, 2006 and which issued on October 27, 2009 as U.S. Patent No. 7,608,847, which claims the benefit of and priority to U.S. provisional patent application Serial No. 60/751,371, filed on December 16, 2005.

### TECHNICAL FIELD

The present invention relates in general to radiation protection and, more particularly, to a suspended personal radiation protection system.

### BACKGROUND OF THE INVENTION

Radiation is used to perform many medical diagnostic and therapeutic tests and procedures. Medical, veterinary, or research personnel may be involved in the performance of these tests and procedures. These professionals are being exposed to scattered radiation as they perform their work. The long-term effects of this exposure are poorly understood at the present time, but are considered serious enough to warrant mandatory protection for operators, who are required to wear garments or barriers that contain materials, which absorb a significant proportion of the radiation. In order to properly perform tests or procedures on patients, operators require freedom of motion. Providing a personal radiation protection system and method that properly protects operators, while allowing operators to move freely and comfortably, presents a significant challenge for operators in radiation environments.

US2009/0184269 discloses a system for providing radiation protection that comprises a garment that protects the operator's body from radiation. The garment is supported by a suspension component that reduces a portion of a weight of the garment for the operator. The garment includes a belt having an release mechanism that offers entry into the garment. The belt opens to allow the operator to enter the garment and the release mechanism is arranged such that the operator can activate the release mechanism without losing sterility.

US2009/0256044 discloses a system and method for suspending a personal radiation protection device. The system includes a support member and a cable mechanically suspended form the support member. The system also includes a member for counter balancing the weight of a personal radiation protection device.

US 5 274 851 discloses a protective garment incorporating radiation resistant material and has a resilient support for conforming to and supporting the weight of the garment across the back of the wearer. By providing resilient back panels which extend form the shoulders the front panel weight is distributed over the shoulders and back. Utilising such a protective garment reduces upper body fatigue for those required to wear such garments.

### SUMMARY OF THE INVENTION

According to a first aspect of the invention there is provided a protection apparatus having all the features set out in claim 1 of the appended claims.

According to a second aspect of the invention there is provided a method of protecting an operator form radiation comprising the steps set out in claim 10 of the appended claims.

A method, a system, and an apparatus for implementing a suspended personal radiation protection solution are provided that substantially eliminate or reduce the disadvantages and problems associated with previous approaches.

According to the present invention, a system for offering radiation protection includes a garment that contours to an operator's body. The garment protects the operator from a substantial portion of radiation which is scattered about during a treatment or testing procedure. The garment is supported by a frame connected to a suspension system that reduces a portion of the garment weight on the operator.

According to one series of embodiments of the invention disclosed herein, the suspension system may include jib cranes for supporting the personal radiation protection system. Jib cranes typically utilize an arm that rotates around a post in the horizontal plane. A trolley can move along the arm of the jib, and anything may be suspended from the trolley, such as the radiation protection device and/or a balancing mechanism. When combined with a balancing mechanism to counteract the weight, it thus allows freedom of motion of the suspended object in the X,Y,Z spatial volume defined by the arc of the arm's rotation. The base may be attached to a ceiling, floor, wall, portable stand, portable table or other fixed object. Many variations or enhancements upon the above described basic system can occur with jib systems or other suspensions components as disclosed herein.

According to one series of embodiments of the invention disclosed herein, the suspension component comprises a reaction arm, manipulating arm, balancing arm, articulating arm, torque arm, and/or other rotating-jointed, articulating, mechanically assisted manipulator. In these embodiments, the garment is attached to a frame which is directly and rigidly secured to such articulating suspension component or components. This is in contrast to embodiments of related applications where the frame is suspended by rope or wire, which, in some circumstances, may undesirably introduce slack, suffer from delayed suspension component movements, or even cause backlash, which is a common problem encountered in any tethered arrangement.

According to the present invention disclosed herein, a binder system is provided which allows the operator to enter the radiation protection system, remain in proximity to the protection system while conducting a test or procedure, and then exit from the protection system without the loss of sterility.

According to an embodiment of the present invention, the binder system comprises a ball and cup attach/detach mechanism. A harness or belt is secured to the operator. The harness or belt includes a binding component, such as a spherical ball capable of being mated with a corresponding binding component such as a cup or socket attachment point affixed to the radiation protection system. The operator positions the ball in appropriate proximity with the cup resulting in the ball and cup mating thereby detachably securing the operator to the radiation protection system. When the operator desires to disengage from the radiation protection system, the operator need simply overcome the relatively small coupling force maintaining the mated connection between the ball and cup connection without losing sterility. Alternative embodiments of a binding system are disclosed herein which include the use of mechanical binding systems, friction binding systems, magnetic and electromagnetic binding systems as are commonly known in the art.

According to another embodiment of the present invention, the radiation protection device includes a suspension assembly with an extension arm. The extension arm may also include a counterbalance or tension mechanism to offset the weight of the garment, face shield and frame supporting the garment. The extension arm improves the range of motion afforded to the operator while using the personal radiation protection device and the responsive motion of the suspension component to the movements of the operator.

According to another embodiment of the present invention, the radiation protection device includes a configurable face shield. The face shield may include an adjustable front plate and adjustable side plate attached to the frame and configured to provide optimal shielding to the operator's face and neck area. The face shield is securably detachable to the frame, and while attached to the frame, the front plate and/or side plate may be independently oriented into an appropriate position relative to the operator. The side plate may be removed, inverted, or placed on the right or left side of the front plate, so as to provide optimal protection to the operator's head and neck while performing tests or procedures.

According to another embodiment of the present invention, the radiation protection device includes a lighting component, or lamp. The lighting component may be attached to the suspension assembly or frame as desired by the operator or the demands of the environment where the radiation protection device will be utilized. The lighting component may be mounted so as to be adjustable and may be configured to maintain relatively constant orientation with the operator. Adjustments may be possible by the operator by manipulation of a sterile handle or a sterile draped handle. Depending on the mounting location of the lighting component, the weight of the lighting component may be used as a counterbalance to advantage the mechanics of the system.

According to another embodiment of the present invention, the radiation protection device includes an instrument holder for holding instruments, tools or other objects which are used by the operator during tests or procedures. The instrument holder may be detachably secured to the suspension assembly, frame or garment. The instrument holder may be a tray, a pouch or a magnet, or any combination of such, which acts to hold instruments or other objects as desired by the operator.

According to another embodiment of the present invention, the radiation protection device includes environmental controls for adjusting and maintaining a comfortable environment for the operator while utilizing the radiation protection system. Environmental control mechanisms which may be detachably secured to, or rigidly attached, to the radiation protection system include fans or air blower systems to provide cooling to the operator. Thermoelectric heating elements located in close proximity to the garment may also be integrated into the personal radiation protection system to provide a heat source to the operator as known in the art.

In accordance with another embodiment of the present invention, the suspension apparatus connects to and supports the shield and/or garment device about its center of gravity or about a point or points in close proximity to the shield and/or garment's center of gravity. This improves motion with regard to certain operator movements, such as bending forward or sideways. Suspension of the shield and/or garment about its center of gravity, or substantially close to the center of gravity or in the coronal (frontal) plane containing the center of gravity, can be accomplished using a ball-in-socket joint along the garment frame, or by pivoting the garment frame about strategically placed axles or pivot joints.

In another embodiment of the present invention, a non-overhead means of suspension is discussed. The frame is supported at or near its center of gravity by a balancing arm, which is itself rotatably secured about the upright post of a floor-based stand. Floor-based docking and ceiling-based docking are possible as depicted herein. Variations of the floor-based stand may include: a mobile floor stand with wheels and counterbalancing weights; a mobile floor stand with an anchoring means for locking the floor stand in place; or, a mobile floor stand that is stabilized by a stationary ceiling post.

Another alternative embodiment of the present invention includes a portable, floor-based, shield and/or garment-suspending back table. Such a back table can comprise a portable track stand which allows lateral sliding movement of a balancing arm for suspending the radiation shield/garment. Alternatively, the shield/garment can be suspended by a table-mounted manipulator arm, articulating arm, reaction arm, or balancing arm which can be rotatably secured to the back table by a table-mounted upright post. Several means for securing the back table to the floor are disclosed, including: stowable floor hooks, floor rings, lockable table wheels, and/or counterbalancing weights or a combination thereof.

Other technical advantages of the present invention will be readily apparent to one skilled in the art from the following figures, descriptions and claims. While specific advantages and embodiments have been enumerated above, various embodiments may include all, some, or none of the enumerated advantages.

### BRIEF DESCRIPTION OF THE DRAWINGS

The novel features believed characteristic of the invention are set forth in the appended claims. The invention itself, however, as well as a preferred mode of use, further objectives and advantages thereof, will be best understood by reference to the following derailed description of illustrative embodiments when read in conjunction with the accompanying drawings, wherein:
**FIGURE 1** is a perspective view of a suspended personal radiation protection system in accordance with the present invention;
**FIGURE 2** is a close up perspective view of a personal radiation protection system in accordance with the present invention;
**FIGURE** 3 is a perspective view of a harness component of the binding system in accordance with the present invention;
**FIGURE 4A** is a side view illustrating the ball and cup binder system in accordance with the present invention;
**FIGURE 4B** is a side view illustrating an alternative ball and cup, or finger binder, system in accordance with the present invention;
**FIGURE 4C** is a side view showing an alternative binding system utilising a ball and cup binder system in accordance with the present invention;
**FIGURES 4D** and **4E** are perspective views showing an alternative integrated magnetic and mechanical binder system utilizing magnets and interlocking blocks and fingers shown in separated and interlocked positions, respectively, in accordance with the present invention;
**FIGURE 4F** is a perspective view of the magnetic mechanical binder system integrated with a harness on the user and with the personal radiation protection system in accordance with the present invention;
**FIGURE 5A** is a side view of an extension arm in accordance with the present invention;
**FIGURE 5B** is a side view of another embodiment of an extension arm showing the extended extension arm in accordance with the present invention;
**FIGURE 5C** is a perspective view depicting an extension arm with a tension mechanism providing a balancing force for the personal radiation protection system in various spatial positions in accordance with the present invention;
**FIGURE 6A** is a front view of the face shield in accordance with the present invention;
**FIGURE 6B** is a top view of the face shield in accordance with the present invention;
**FIGURE 6C** is a perspective view depicting the hanger, face shield and frame in accordance with the present invention;
**FIGURE 6D** is a perspective view showing the face shield attachment and adjustment mechanism attached to the frame in accordance with the present invention;
**FIGURE 6E** is a perspective view showing an alternative face shield attachment and adjustment mechanism in accordance with one embodiment of the present invention;
**FIGURE 6F** is a perspective view showing the adjustable features of the face shield in accordance with one embodiment of the present invention;
**FIGURE 6G** is a perspective view depicting an alternative embodiment of the adjustable face shield in accordance with the present invention;
**FIGURE 7** is a perspective view depicting a lighting component affixed to the personal radiation protection device in accordance with one embodiment of the present invention;
**FIGURE 8** is a perspective view of a manipulator arm suspension system integrated with a bridge and trolley system in accordance with one embodiment of the present invention; and,
**FIGURE 9** is a perspective view of a manipulator arm suspension system attached to a fixed support with an alternative shield system in accordance with one embodiment of the present invention.
**FIGURE 10** is a perspective view of a suspended personal radiation protection system in accordance with the present invention;
**FIGURE 11** is a perspective view of a personal radiation protection system in accordance with the present invention;
**FIGURE 12** is an elevated side view of the manipulator-arm/reaction-arm-type suspension system in accordance with the present invention;
**FIGURE 13** is an elevated side view of an alternative suspension system in accordance with the present invention;
**FIGURE 14** is an elevated side view of an alternative suspension system with a manipulator arm in accordance with the present invention;
**FIGURE 15** is a perspective view of the wrist of the manipulator arm seen in **FIGURE 14****,** in accordance with the present invention;
**FIGURES 16A-16D** depict the functionality of the center-of-gravity attachment mechanism in accordance with the present invention;
**FIGURES 17A-17B** offer perspective views of a ball-and-cup center-of-gravity attachment system of the frame to a suspension system in accordance with the present invention;
**FIGUREs 18A-18D** offer perspective views of axle-based center-of-gravity attachment of the frame to various embodiments of alternative suspension components in accordance with the invention disclosed herein;
**FIGURE 19** is a perspective side view of a portable, floor-based, non-overhead suspension system in accordance with the present invention;
**FIGURE 20** is a perspective side view of the portable, floor-based, non-overhead suspension system of **Figure 19** modified for floor-based docking, in accordance with the present invention;
**FIGURE 21** is a perspective side view of the portable, floor-based, non-overhead suspension system of **Figure 19** modified for ceiling-based docking, in accordance with the present invention;
**FIGURE 22** is a top view of a portable, floor-based, back-table-mounted garment suspension system, in accordance with the present invention;
**FIGURE 23** is a perspective view of an embodiment of a portable track stand for use with the back-table-mounted garment suspension system in accordance with the present invention;
**FIGURE 24** is a perspective view of an embodiment of a portable track stand for use with the back-table-mounted garment suspension in accordance with the present invention;
**FIGURE 25** is a perspective view of an embodiment of the stowable, recessed floor hook in accordance with the present invention;
**FIGURE 26** is a perspective view of an embodiment of a portable track stand for use with the back-table-mounted garment suspension system in accordance with the present invention;
**FIGURE 27** is a top view of the range of motion available to the portable track stand for use with the back-table-mounted garment suspension system in accordance with the present invention;
**FIGURE 28** is a perspective view of the portable, floor-based, back-table-mounted, non-overhead or overhead garment suspension system where an example table-mounted manipulator arm is depicted in an operating position and a parked position, in accordance with the present invention; and,
**FIGURE 29** is a perspective view of the portable, floor-based, back-table-mounted overhead jib system with extension arm on the trolley in accordance with the present invention.

### DETAILED DESCRIPTION OF THE INVENTION

For purposes of teaching and discussion, it is useful to provide some overview as to the way in which the invention disclosed herein operates. The following information may be viewed as a basis from which the present invention may be properly explained. Such information is offered for purposes of explanation only and, accordingly, should not be construed to limit the broad scope of the present invention and its potential applications.

Radiation is used to perform many medical diagnostic and therapeutic tests and procedures. The human patient or animal is subjected to radiation using minimal doses to enable completion of the medical task. Exposures to radiation are monitored to prevent or reduce risks of significant damage. Medical, veterinary, or research personnel may be involved in the performance of such procedures in great numbers.

Over many years, these professionals are being exposed to scattered radiation as they perform their work. Although their daily exposure is generally less than that for the patient, there are adverse cumulative effects to the operators. These long-term effects are poorly understood but are considered serious enough to warrant mandatory protection to workers in the form of garments or barriers that absorb a significant proportion of the radiation. There is a wide variety of such barriers commercially available, but these solutions have significant limitations for the operators who must come in close contact with the subject. These operators may be physicians and their assistants, or technically skilled medical personnel, who perform simple or complex medical procedures using their bodies and hands in proximity of the patient. In many cases, scatter radiation from the subject or physical elements in the direct radiation beam will pose significant health risks and unacceptably high exposure.

Risks of radiation exposure at the levels of medical personnel include cancers, cataracts, skin damage, etc. A review of current protective systems outlines their limitations. Radiation-absorbing walls are useful to contain the radiation to a room, but do not prevent exposures within their confines. Barriers within the room, such as floor or ceiling supported shields, are effective at blocking radiation for personnel who are not in close contact with the radiation field, such as some nurses and technologists, but must be positioned or repositioned frequently when personnel move around the room. They also provide cumbersome interference for operators performing the actual medical procedure. They may also be difficult to keep sterile when attempting to use them within the sterile field.

The most commonly used protection for operators involves the use of garments containing radiation-absorbing materials, generally lead or other metals, which are worn in the fashion of a coat, smock, skirt, or vest, and do not contaminate the sterile field because they are worn underneath the sterile covering gown. These garments are heavy and uncomfortable, and their long-term usage is known to be associated with diseases of the spine, especially the neck and back, knee disorders, and other musculoskeletal problems, which can result in disability, medical expenses, and decreased quality of life for the operator.

The trade-off between protection and garment weight results in the frequent use of garments that do not cover the legs, head, torso, and eyes optimally, and may provide sub-optimal radiation protection due to the thickness of the metallic material being limited by the tolerability of the operator. To protect other radiation-sensitive tissue such as the corneas of the eye and the thyroid, special heavy glasses containing metallic compounds and a collar around the neck are often worn. Even when the operator is encumbered with these items, the base of the skull which may contain sensitive bone marrow and the face are still unprotected. Personal face and neck shields address this problem, and are commercially available, but are rarely worn due to their cumbersome nature and heavy weight.

Such problems have been present for many years with various attempts to solve them. For example, modifications to floor-supported mobile shields attempt to provide improved dexterity for the operator relative to the standard bulky mobile barrier, and a floor support system with a modified garment design also attempts the same. However, they still act as obstacles to the free movement of the operator using them. Another system of barriers (such as those referred to as radio-protective cabins) around the patient has been proposed, but that appears cumbersome, confining, and inhibitory to operator movement both gross and fine, patient/subject contact, and sterile field operation.

Ceiling mounted barriers around the patient also limit contact between patient and operator, and may make control of a sterile field difficult. One configuration includes a ceiling mounted device, which supports the weight of a lead garment, involving a dolly, or trolley, movable in one linear axis, with or without an extension arm that rotates around a central point on the dolly. Such mechanical configurations are in place for other types of suspended barriers and their motion mechanics may not be well suited for use with something attached to the operator's body since the operator must frequently move rapidly and freely in all three spatial axes. Typically, the operator will walk in unpredictable and rapid patterns over an operating area. One configuration includes the garment being suspended by a simple expansion spring, which will provide uneven forces on the shield and operator depending on its degree of expansion occurring with operator motion due to the nature of its simple spring mechanics. It may also result in harmonic motions that affect operator dexterity. In addition, failure of the spring due to cycle stresses could lead to operator injury. Likewise, location of the spring in a vertical direction above the operator results in movement limitations due to ceiling height. Integration of the system with the heavy image intensifier monitor screen would further encumber the operator from normal motion.

A discussion of the types of motion performed by operators during their work is relevant. Operators generally stand next to an operating table where the patient is positioned. They often reach over the patient to various parts of the body, and they may lean forward while reaching for items, surfaces, etc. This puts stress on the operator's spine when heavy garments are worn. They may bend or stoop, but usually only to a small degree because the workplace containing the patient limits vertical motion. In addition, most procedures involve a sterile field where the operator's hands, arms, and torso must remain confined, so excessive vertical motion is prohibited. The operator may need to move considerably in all three spatial planes by walking or turning their body. Clearly, the operator requires freedom of motion in each of these directions to perform a procedure or test.

Overhead cranes have been available for many years and are commonly employed in the materials-handling industry. The following is a description of a bridge crane. A bridge crane includes at least one bridge, and a trolley moving on the bridge, end trucks arranged at the ends of the main bridge to support the main bridge, and wheels arranged to the end carriages intended to move along substantially parallel rails substantially parallel to the end trucks. In some embodiments, articulated end trucks provide increased maneuverability for the bridge so as to allow the bridge to rotate or sway, while staying safely attached to the rails. Smaller cranes such as those to be used to support a load up to 250 pounds are often operated by workers without the aid of motorized assistance because the crane's movable parts are light enough to be manipulated by hand. Different systems are employed to suspend the load from the cranes, including hoists, balancers, and intelligent assist devices as known in the art.

Tool balancers are also currently available and help to suspend tools in the workspace in a manner that provides ergonomic benefits for the workers using them. The tool balancer is generally attached over the workspace, and reels out cable from which the tool is suspended. Adjustments may be made to provide a "zero gravity" balancing of the tool at the desired height such that the worker may move the tool up or down within a working range without having to bear a significant portion of the tool's weight. Adjustments may cause the tool balancer to exert a stronger upward force such that the operator must apply a downward force on the tool to pull it down to the workspace and the balancer will cause the tool to pull it down to the workspace and the balancer will cause the tool to rise when the operator releases it.

Tool balancers may be of the spring, hydraulic or pneumatic variety, referring to the force mechanism, which provides the balancing force for its operation. A spring tool balancer, such as in one embodiment of the invention disclosed herein, generally contains a coiled flat spring, which is attached to a reel with a conical shape and which serves as the platform for the winding of the cable. The conical shape provides a variable mechanical advantage, which offsets the variance of the force provided by the spring as it winds or unwinds. The result is a relatively constant force on the cable within a definable working range.

Safety concerns mainly involve falling objects, strength of the suspension device, strength of the cable, and operator falls. The balancer can be attached to the trolley by its own hook and a safety chain. The suspension device is commercially available at specified maximum loads, which include a wide safety margin. The mounting of the suspension device is accomplished according to architectural/engineering requirements and standards for suites where the present invention is to be utilized.

Tools or other loads may also be balanced by arms that provide torque approximately equal to the weight of the load via various mechanical systems including internal cables and pulleys with springs, pneumatic force, or counter-weights. Such arm systems could be manipulator arms, torque arms, reaction arms, or balancing arms as are known in the art.

Detachment of the frame and/or garment from the suspension system will require certain care. A cable stop on a conventional tool balancer will prevent the hanger from going higher than the set level. Some balancers are equipped with a locking mechanism that prevents motion of the cable during load change or removal. This permits simple removal or exchange while standing at ground level. Alternatively, without activating a locking mechanism, the operator could raise the load upwards until it contacts the balancer stop, or until the arm is fully raised to its capacity in the case of an arm system, and then remove the garment without concern for sudden upwards, uncontrolled motion of the balancer cable or arm and attached components. Alternatively, a weight, which is approximately equivalent to the weight of the supported system, could be attached to the cable, rod, or arm prior to disengaging the supported system. This would act to "drop" the supported system and require it to be supported by the operator, who may then disengage it from the cable, rod or arm system. The weight will prevent any upward motion of components in an uncontrolled manner. The next time the frame and/or garment is attached, the weight could be removed after secure attachment of the supported load is confirmed.

For most operational scenarios, the protection system need not be detached from the suspension system (e.g. cable, rod, or arm supporting the system). In alternative embodiments, it may be left suspended and simply moved out of the way of other activities. Another alternative method would involve setting the force on the tool balancer or balancing arm to be slightly greater than the weight of the suspended shielding components. Once removed from the body, the suspended protection system would then slowly and safely rise up until stopped by the stop mechanism. Upon next use, it could be easily pulled back down into position by the operator. Annual inspections of the system may be performed for cable frays, hook or lock malfunctions, and suspension component flaws.

In the event of an operator fall, it is unlikely that the system will contribute to operator harm since in some embodiments the suspension system allows the operator to reach the floor without restriction. According to the invention, the design of the system provides for the safe and quick detachment of the operator from the radiation protection system. The binder system quickly provides engagement/disengagement from radiation protection system as the binding forces keeping the operator in proximity to the radiation protection system would be easily overcome by the forces exerted during a fall.

In the event of malfunction, many support systems are equipped with automatic locking mechanisms to prevent dropping of the load supported by the support system. In the event of actual detachment, the frame supporting the suspended shielding components may be designed such that there are pads positioned over the shoulders of the operator which would gently engage the operator's shoulders to support the weight of the device in the event of a suspension failure. However, this type of malfunction would be rare as it would generally be avoidable with adequate support structure strength and annual inspections of the entire system.

In the event that rapid detachment of the operator from the system is necessary due to emergency, the binding system disclosed herein is designed to provide simple and quick disengagement between the operator and the radiation protection system. As disclosed further herein, a simple hand push or wave or actuation of a switch by the operator or another in various embodiments results in the operator disengaging quickly and safely from the radiation protection system without detachment of the garment from the system. The garment can be left hanging on the system and then moved clear of the patient or stretcher. Likewise, the operator can quickly disengage from, and then reengage with, the system while remaining sterile.

Turning back now to the general problem of radiation, it is evident that people are often exposed to radiation in the course of their work. The proposed concept, outlined herein, describes a device and technique intended to address many of the aforementioned problems. It provides substantial shielding for the operator: covering a large part of the body. The shielding capacity can be increased with thicker layering of heavy metal or other radiation-protective material, thus reducing the stress on the operator because the device is substantially weightless to the operator. The device is close to the body of the operator, much like a conventional apron, but it is not supported by the operator. It moves with the operator as he/she moves around within the working field and sterile field, and allows movement of arms and body parts to accomplish the procedure at hand.

The overall benefits of the device include: improved comfort for the operator who is no longer supporting heavy-shielding clothing, improved radiation protection to an operator through a much greater portion of body shielding compared to a conventional apron, as well as more effective shielding of much of the covered body parts due to greater use of the shielding material. This approach also offers a musculoskeletal benefit due to the absence of a significant weight burden on the operator.

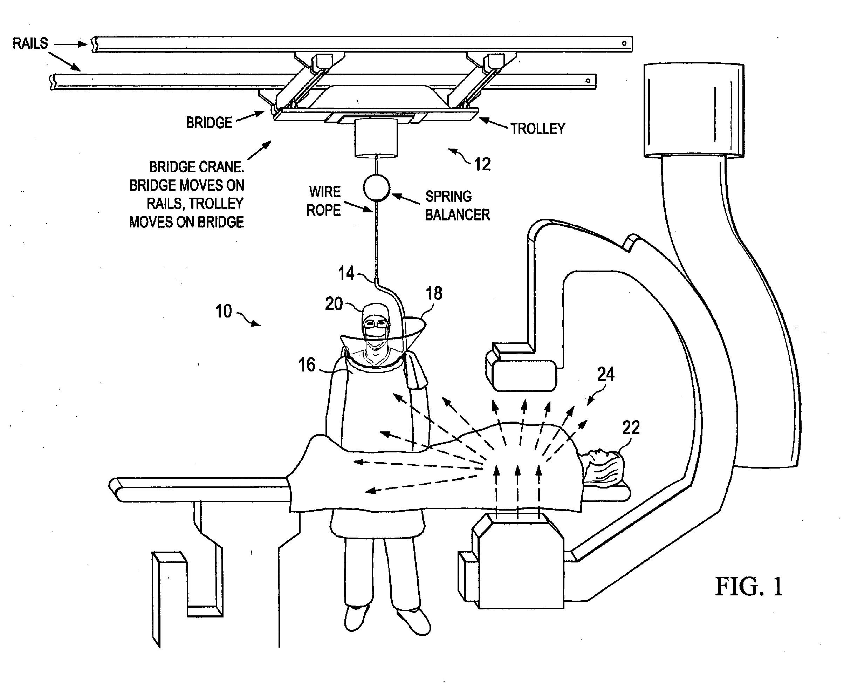

Turning now to the figures, Figure 1 is a perspective view of a radiation protection system 10. System 10 includes a suspension assembly 12, a frame 14 for supporting a radiation protection garment 16, and a face shield 18 in the embodiment shown. Operator 20 utilizes system 10 for the purposes of providing protection to the operator during a procedure performed on patient 22 using radiation rays 24. The protection system 10 may be easily installed or integrated into a typical operation suite.

In general, the garment 16 is suspended from the suspension assembly 12 and supported by frame 14. Frame 14 contours around the shoulders, chest and torso of the operator 20 and supports the garment 16 and/or face shield 18, along with other potential devices or instruments such as an instrument tray, lighting apparatus, environmental control or other instrumentation which may easily be integrated with system 10 as described hereafter. In one embodiment shown in **Figure 1****,** frame 14 is connected with the suspension assembly 12 wherein the suspension assembly may include a rigid rod, a wire rope, an extension arm, a manipulator arm, or other suspension means discussed herein. Multiple wire ropes, reaction arms, tool balancers, trolleys and bridges, and other various support systems as are known in the art, may be utilized for supporting the radiation protection systems and embodiments shown herein as appropriate and desired by the user or facility where the system will be utilized.

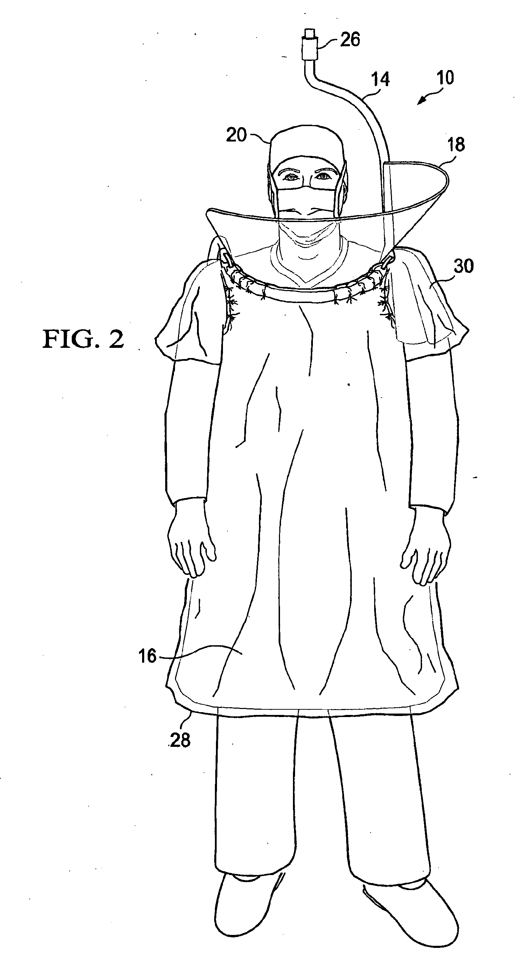

**Figure 2** further illustrates the frame and garment architecture. The protection system 10 is shown with frame 14 which contours around the neck, shoulders and upper torso of the operator 20. In one embodiment, the frame 14 is open in the back to permit rear entry into the system 10 by the operator 20. Frame 14 supports garment 16 and is detachably connected to the suspension system 12 (as shown in **Figure 1**) via hanger 26. It should be noted that hanger 26 may be an attachment mechanism using attachment means as known in the art, including but not limited to, threaded connectors, hook and rings, or and/or screw type connectors. Hanger 26 may also be a rigid, rotatable or swiveling connector as required by the operational mandate for the system 10. In still other embodiments, the frame 14 includes an attachment means for connection to the suspension assembly 12, without the requirement of hanger 26. The depicted embodiment allows the operator 20 to position herself in proximity with, or behind, the garment 16 such that the operator 20 is not supporting the weight of the garment 16 or frame 14. In this sense, she is liberated from the typical and problematic weight constraint of typical radiation garments and protection systems. The frame 14 and garment 16 are constructed to allow unimpeded rear entry to the radiation protection system by the operator 20 without the need to manipulate the frame 14 or the garment 16 to engage with the system. In other embodiments, frame 14 may be constructed as a shaped, rigid unit or may be constructed of semi-rigid, flexible materials allowing the operator 20 to manipulate the shape of the frame 14 to suit her body shape. Manipulation of frame shape can also be provided by the use of rigid frame materials connected by joints of many different types including torque hinges, or friction hinges, which are somewhat similar to conventional door hinges with friction in their movement, often designed to specifications or adjustable, that enable the frame to keep the desired shape, once set, until sufficient force is again applied to overcome the friction. According to the invention, the operator 20 will engage with system 10 using a binding system disclosed herein The invention herein is different from conventional aprons or vests and previous suspended aprons which have more conventional shoulder constructions that required the garment arm hole to be opened up somewhat or lifted or manipulated in some way for the operator to gain entrance. This can be a problem with sterile entry and exit of an operator wearing a sterile gown and trying to engage a device wrapped in a sterile drape. The rearward openness of system 10 aids in these maneuvers without contamination of the sterile operator or field which is another novel aspect of the invention disclosed herein.

Some embodiments of system 10 disclosed herein are shaped to require no user manipulation and do not require a change in shape to enter, use, and exit from the system. In various alternative embodiments, the frame 14 and garment 16 may be shapeable, which can be accomplished with or without the presence of the operator's body. Some of the unique features of the invention disclosed herein spring from the novel frame 14 which is generally positioned in front of the operator 20 during use. Frame 14 may be constructed from rigid, jointed, semi-rigid and/or flexible material to allow frame 14 to be shaped or to be fashioned to substantially contour with the operator 20. In one embodiment, frame 14 is semi-rigid and can be shaped to substantially contour to the body of a particular operator. When the system 10 is used by another operator 20 of different body size, the frame 14 may be reshaped to adjust the contour to the body size of the next operator 20. As depicted in Figure 2, the frame 14 sweeps in front of the operator 20 from one side to the other, acting as a support for the garment 16 and providing form and structure to the garment 16 in the areas near the operator's head, shoulders, arms, and upper body, where important movement occurs during a procedure. It also serves as a mechanism to assist in the proper balance of the entire system 10 so as to maintain proper operational orientation. Previously described suspended protection systems typically attach at the shoulders of the operator without providing a frontal frame element that performs the functions described herein. Frame 14, combined with the disclosed suspension system 12 and various other support alternatives discussed herein, allows concentration of the mass and weight of the system 10 towards the front and sides of the operator 20, while maintaining proper balance and orientation relative to the operator 20. This facilitates simple and effortless entry and exit of the operator 20 from the system 10. It also permits secure attachment of a face shield 18 in front, and to the sides if needed, of the operator 20 while maintaining proper balance and orientation, without restricting the motion of the operator's body and arms when using the system. The shape desired by the operator 20 can be substantially maintained regardless of the operator's presence in the system, except allowing for some minor flexing of the arm flaps 30 and fabric as the operator 20 moves. However, no substantial shaping or change in form is necessary for the operator 20 to enter the device 10, thus facilitating sterile entry/exit. Also, a sterile drape 28 may be used to substantially cover garment 16 and portions of frame 14. Sterile drape 28 is easily inserted over the desired portions of garment 16 and frame 14 to provide additional sterile protection.

Another novel improvement disclosed herein, is that in some embodiments a circumferential belt or strap is not required to be fastened behind, around or in front of the operator; the operator is never completely surrounded by any continuous component as in conventional aprons and protection systems which use a belt that joins the apron between the shoulder blades or elsewhere to keep it on the operator. The invention disclosed herein may include embodiments that wrap completely around the operator's torso. Nonetheless and although the rear components may touch or overlap in some cases, some embodiments are maintained in this position by frame elements that are oriented so that opening and closing of the frame to allow entry of the operator does not require manipulations near the back of the system or operator, or the passage of any straps or belts around the rear of the system or operator. The invention disclosed herein includes embodiments that eliminate this need by providing a frame structure that permits engagement to the operator without requiring a change in the frame shape or manipulation of belts or other components each time the operator engages or disengages from the system. In some embodiments, the operator is never surrounded by a component that must be disengaged upon entry or exit.

Additional alternative embodiments are envisioned wherein the rear frame or apron elements connect to each other, but this connection does not require manipulation of rear components, or passage of operator's or assistant's hands towards the rear. Such a previously described embodiment utilized a frame belt with a magnetic component in the rear that facilitated a binding of the rear components together once they are moved together in proximity to each other using manipulations performed at the sides or front. Although some alternative embodiments may include a flexible belt or tie that connects the rear frame or apron elements in the rear, or wraps around the body in a manner similar to a kitchen apron or conventional clothes garment, many alternative embodiments are described that permit partial or complete circumferential enclosure of the frame or apron around the operator that eliminate the need for manipulation or passage of the hands rearward, thus facilitating rapid entry and exit, and more importantly facilitating the ability of an operator wearing a sterile gown to enter and exit the device that is also protected by a sterile cover, in a manner where sterility is not broken by the passage of non-sterile objects, or use of objects that are difficult to drape, or the use of an assistant or the passage of operator's hands out of the conventional sterile field.

Frame 14 provides a shape for the garment of generally an arc or curve that accommodates the shape of the operator's body, whereas previous known suspended garment systems assumed their contoured shape upon engagement with the operator, by being flexible without any predetermined shape, and without the ability to maintain the working shape in the absence of the operator's body. Previous suspended garment systems were basically suspended clothing, like an apron or shirt that were donned in similar manners to corresponding clothing articles, whereas the device disclosed herein utilizes a shaped rigid, or flexible in other embodiments, frame 14 to which flexible garment 16 material may be attached to allow the maintenance or molding of the frame to an appropriate and comfortable shape so that the operator 20 can simply step into it, although in alternative embodiments the garment 16 material may be stiff or nonmalleable. The frame 14 may be attached to the suspension system 12 at one or multiple points as disclosed herein. In one alternative embodiment shown in the figures, unilateral suspension provides an unobstructed contra lateral side, while providing a more substantial support structure for greater strength. Additionally, while using radiation during a procedure or task, the operator 20 may freely move in the X, Y and Z spatial planes such that the frame 14, garment 16 and face shield 18 are substantially weightless to the operator 20.

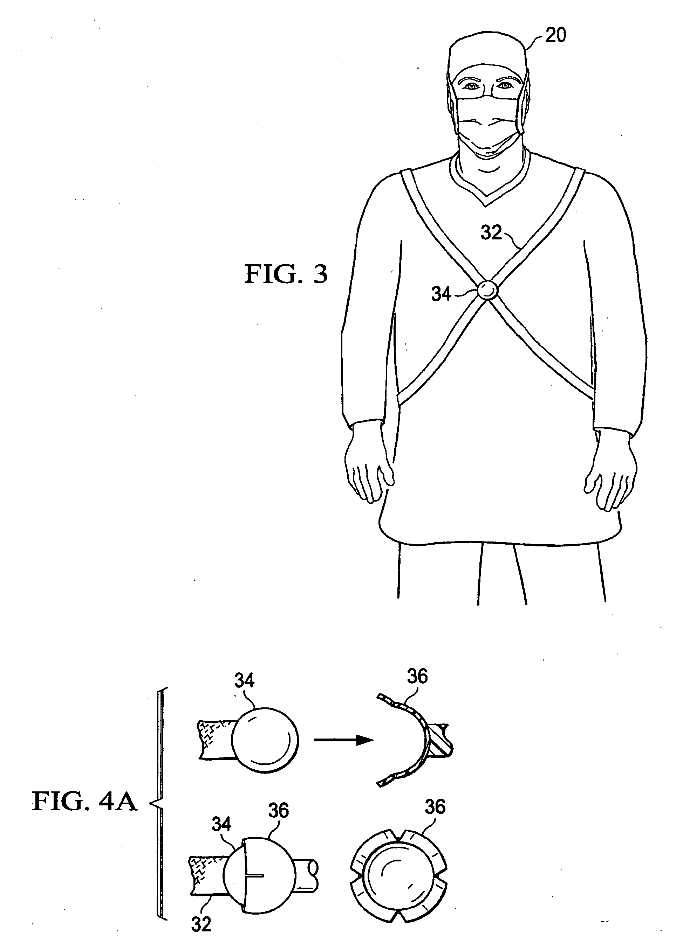

Figure 3 depicts one embodiment of an engageable/disengageable binding system which is utilized for keeping the operator and the radiation protection system linked or connected in relative proximity together during a procedure. This embodiment depicts an operator 20 who has donned a harness 32, which may consist of a belt, vest, strap, garment or other structure capable of being detachably secured on or about the operator's person. Many different configurations are possible, including an embodiment that incorporates a wide waist belt that provides lumbar support. A first binding component, ball 34, is secured to a location on the harness 32, with a second binding component (not shown) being secured to the frame, garment, hanger, or other attachment point to the radiation protection system as described herein. The harness 32 may be donned over the operator's sterile gown, or underneath the gown, as required. When the first binding component is engaged with the second binding component (see **Figures 4A-4E****)** via friction, magnetic, electromagnetic, or by other mechanical binding mechanisms, the operator is secured to the personal radiation protection system so as to allow the operator and personal radiation system to move freely together in the X, Y and Z planes while maintaining operator proximity with and protection by the system. The binding mechanism may be designed and constructed to accommodate layers of fabric or plastic drape materials that may be present on the operator, such as a sterile gown, and on the device, such as a sterile drape. The binding mechanism(s) would bind together despite the interposed layer(s) without tearing or damaging the layers. When the operator disengages the first and second binding components, both component surfaces would remain covered by the drape, which would remain sterile since they were only in contact with other sterile materials. If sterilized binding components were used outside of the sterile drape, they would likewise remain sterile after disengagement.

**Figures 4A-4E** depict various embodiments of binding systems that serve to engage the radiation protection system to the operator so that it moves with her during operations. Depicted embodiments include various male-female connection mechanisms whereby the male and female components provide mechanical stabilization, flexible and/or rigid fixation between components that are attached to both radiation protection device and operator. In some embodiments, the male-female components also provide a guiding function, which facilitates the proper alignment of the binding components during engagement by the operator.

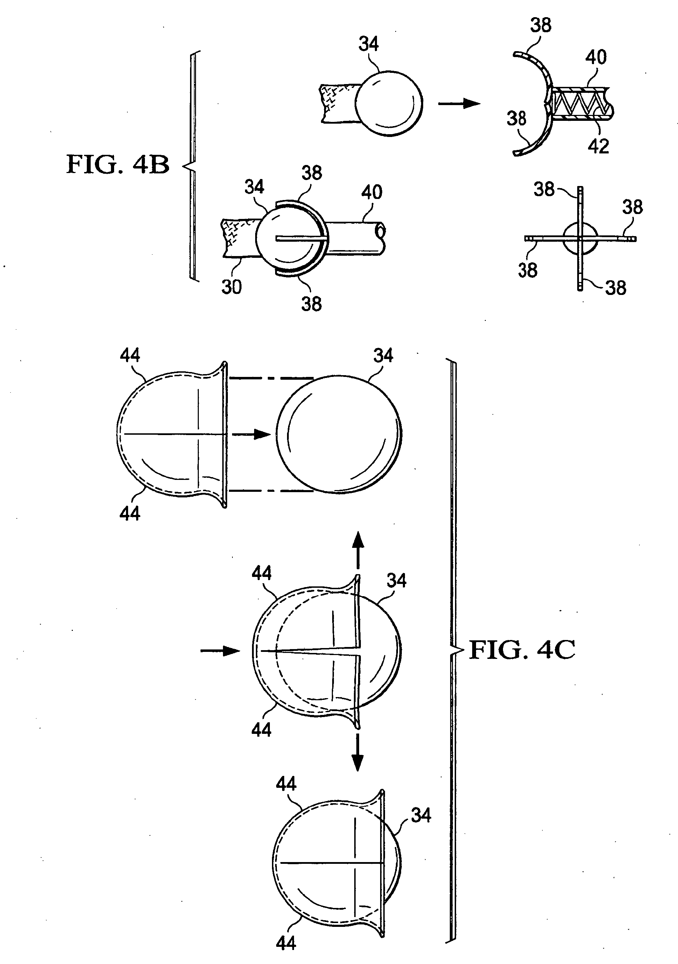

**Figures 4A** and **4B** show one embodiment of a binding system which utilizes a ball 34 and cup 36 connection. As shown in **Figure 4A****,** a first binding component, in this embodiment depicted as a ball 34, is attached to a harness 32 which is donned by the operator 20 as shown in **Figure 3****.** The second binding component, in this embodiment cup 36, is mounted to the garment 16 or frame 14 with the cup 36 shown in the "open" position. Depending on the mounting location of the second binding component cup 36 (e.g. to the frame 14 or garment 16 in various contemplated embodiments), the operator simply moves into relative position and ball 34 is received into cup 36 which to create an engageable/disengageable connection. The ball 34 and cup 36 connection allows the operator and the radiation protection system to move in relative, substantial unison with one another. In alternative embodiments, a variety of geometric shapes for binding components may be constructed so as to mate and have slightly different characteristics, such as multiple attachment sites on the frame, garment and/or operator, or binding components of lower and higher physical profiles as required by design and operational mandates. In various embodiments, the degrees of freedom of motion between the mated binding components may vary from none (such as a rigid connection), to limited within some rotational axes, to complete freedom of movement in all spatial directions. Different kinematic chains of articulation could be constructed to allow translational motion in addition to radial motion, and springs, gas springs, dampers, or other mechanical tensioning means could be employed. The binding system may be constructed so that a release mechanism must be activated to permit disengagement of the operator from the garment and/or frame, for example by operator hand movement or depressing a switch. Alternatively, it could be constructed such that a certain amount of forceful separation or specific rotational or translational movement would overcome the binding forces of the mated binding components and disengage the binding components without activation of other mechanical disengagement systems by the operator's hands or otherwise. This automatic disengagement function could be constructed for routine disengagement, or only as an emergency disengagement function, in one embodiment similar in function to the operation of a ski binding release mechanism. In other embodiments not shown herein, engagement could require activation of a mechanism, via an electronically controlled mechanism, while in other embodiments engagement would occur naturally as the components are pressed together.

In an alternative embodiment depicted in **Figure 4B****,** a second binding component attached to the protection system via rod 40, in this depiction a cup formed with articulating fingers 38 which when introduced into proximity with the first binding component, in this depiction ball 34, act to close around the ball 34, resulting in a flexible and/or rigid secure connection as desired between the first and second binding components. The articulating fingers 38 are held in a closed position forming the connection with the ball 34. In order to disengage from the connection between the ball 34 and cup fingers 38, the operator may activate a release mechanism which overcomes the spring 42 force holding the fingers 38 in the closed position around the ball 34, thus opening the fingers 38 and allowing the ball 34 to become detached, and allowing the operator to quickly and easily exit from the frame or garment. Alternatively, a more rigid connection between the first and second binding components may be fashioned by the use of lock and pin type connectors, magnetic, electromagnetic, key connectors, rigid plates, or other mechanical attachment means as known in the art.

In the embodiment shown in **Figure 4B****,** a spring 42 or other compressive/tensile force system could be used in conjunction with articulating fingers 38 to keep them in a closed cup position. When ball 34 is pressed into the cup formed by fingers 38, the maximum diameter of the ball 34 passes through the smaller diameter of the cup opening, prying the fingers 38 apart against the resistant spring force exerted by spring 42. Once the ball 34 and fingers 38 are in a mated position, the fingers 38 are closed around the ball 34 forming a connection between the first and second binding components.

In an alternative embodiment depicted in **Figure 4C****,** articulating curved cup fingers 44 are shown with rounded lip edges shown on the circumference of the cup fingers 44 may help facilitate the connection process. In this embodiment, a spring mechanism (not shown) is employed to keep the cup fingers 44 in a closed position. Once the ball 34 is pressed into the cup fingers 44 with some force, the cup fingers 44 are pushed open by the ball 34. In this embodiment, the cup is formed by a plurality of cup fingers 44 with curved edges that can open or close as shown. Once the ball 34 has been pushed substantially into the cup formed by cup fingers 44, the cup fingers 44 may then close around ball 34 resulting in a connected binding system. Disengagement between the ball 34 and cup fingers 44 would typically occur in a reverse fashion. The force required to push the ball 34 into the cup fingers 44, or to pull it back out, can be designed to be appropriate for the situation of usage with low effort required by the operator for engagement and disengagement. It is contemplated that many combinations and derivatives of the binding functions discussed herein could be employed with the method and apparatus discussed herein using connection means and mechanisms that are known in the art.

**Figures 4A-4C** depict alternative binding systems utilizing the principles of male and female type components which when joined together create a securably detachable connection. However, it is contemplated that many different shapes, sizes and mechanisms for binding are commonly known in the art and the embodiments disclosed herein are not to be construed as limited as to those which have been disclosed, as such binding mechanisms are too numerous and diverse to be described herein. Likewise, in addition to "press and fit" style components that change their shape to attach and detach, other types of binding mechanisms may be used with the invention herein. For example, friction binding systems, hook and loop binding systems, and simple mechanical binding systems, such as with a hook and ring or snaps, could also be utilized as binding systems allowing for the operator to attach and detach from the radiation protection system.

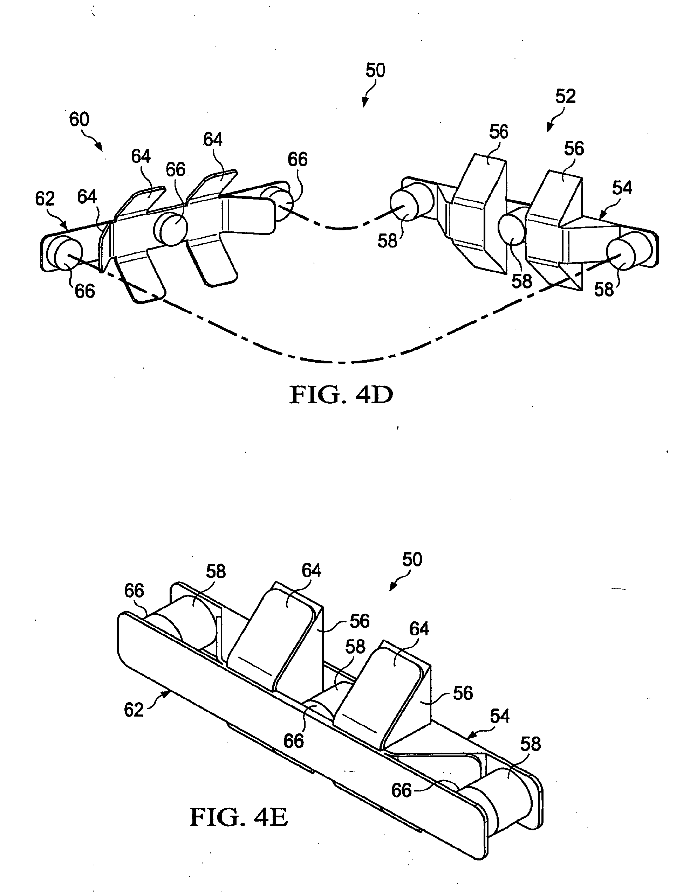

In another embodiment shown in **Figure 4D** and **Figure 4E****,** a magnetic mechanical binding system 50 for binding the operator to the personal radiation protection system is shown. System 50 is composed of first binding component 52 and second binding component 60. First binding component includes base plate 54, one or a plurality of guide blocks 56, and one or a plurality of strike plates 58. Guide blocks 56 and strike plates 58 are secured to base plate 54 via various connection means as known in the art. Guide blocks 56 may consist of a variety of shapes, surfaces and materials with desired characteristics for forming a mating surface with the second binding component 60. The shapes and surfaces may be rigid, deformable, malleable, magnetic, or have multiple physical and mechanical characteristics which assist in forming a connection with the constituents of the second binding component 60. Second binding component includes base plate 62, one or a plurality of guide fingers 64, and one or a plurality of magnets 66. Guide fingers 64 and magnets 66 are secured to base plate 62 via various connection means as known in the art. The shapes and surfaces attached to base plate 62 may be rigid, deformable, malleable, magnetic, or have multiple physical and mechanical characteristics which assist in forming a connection with the constituents of the first binding component 50.

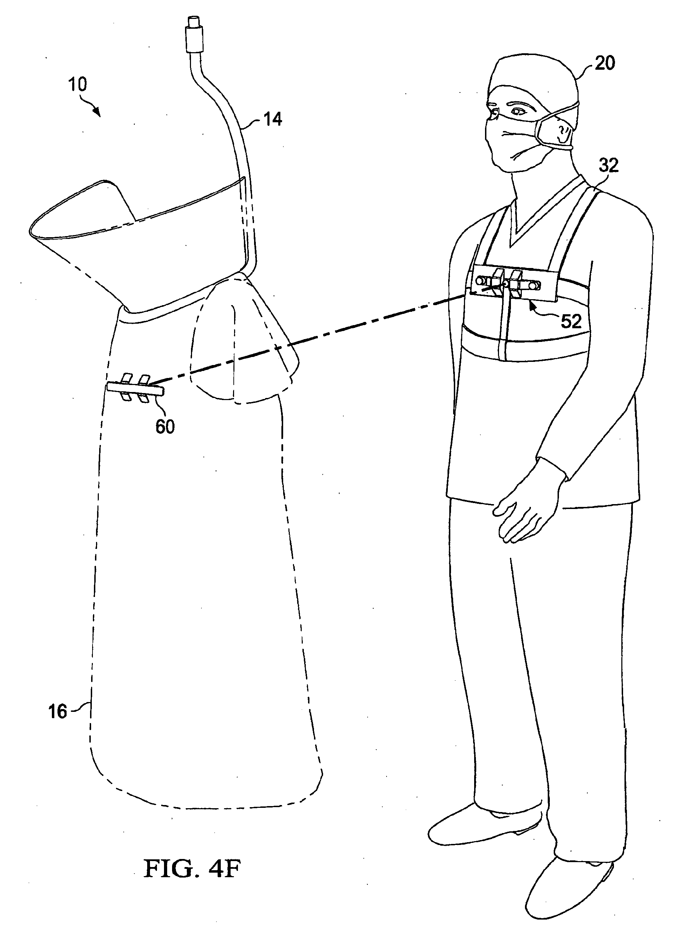

As depicted in **Figure 4D****,** guide fingers 64 orient at angles from the guide blocks 56. The guide blocks 56 and guide fingers 64 assist in guiding the first and second binding components 52, 60 together for forming a binding connection as shown in **Figure 4E****.** Referring to **Figure 4F****,** as the operator 20 moves in proximity toward the radiation protection system 10, the first and second binding components 52,60 may not be on track to line up for mating and forming a secure connection. As such, the operator guides the first binding component 52, which in the embodiment shown is attached to the operator 20 via harness 32, towards the second binding component 60, which in the embodiment shown is secured to radiation protection system 10 via garment 16. Guide fingers 64 are positioned with the guide blocks 56, and as the magnetic attractive force between strike plates 58 and magnets 66 increases, the first and second binding components are mated together to form a sufficient mechanical magnetic binding.

First and second binding components 52,60 are shown in the mated or connected position in **Figure 4E****.** Once engaged, the guide fingers 64 fit securely around in symmetry with the guide blocks 56. This serves to provide an additional mechanical binding component to the binding forces between the operator 20 and the radiation protection system 10, in addition to the magnetic binding forces described in this embodiment. For example, it prevents substantial translational motion of the base plates first and second binding components 52,60 relative to each other. Such translational motion is also resisted by the magnetic force between the strike plates 58 and magnets 66. Thus the magnets 66 do not "slide" against the strike plates 58. Such a combination of magnetic force and mechanical forces is readily adaptable to this embodiment since they resist the inadvertent disengagement of the operator 20 from the system 10 during the performance of procedures. Nonetheless, intentional disengagement from the system 10 by the operator 20 is quite simply accomplished by a sudden, but not excessive, thrusting of the base plates 52,54 directly apart from each other, such as is easily achieved by pushing the radiation protection system 10 away from the operator's body 20, which is a motion that is unlikely to occur inadvertently during normal use. In alternative embodiments, the magnetic binding system disclosed herein may be used without related guide plates, guide blocks or with additional mechanical connection components (e.g. snaps, hook and loop fasteners). In still other embodiments, the first or binding component may simply consist of one or more magnets fixed directly to the operator via a harness, belt or stitched directly to the operator's clothing, with the second binding component consisting of one or more strike plates fixed in a variety of locations on the system 10, including frame 14 and/or garment 14. Other embodiments may use the principles of magnetism or electromagnetism with or without mechanical forces using various shapes of magnets, shapes of guide fingers and guide blocks, or other mating or fastening means components as known in the art.

As shown in **Figure 4F****,** the operator 20 may don a harness 32 with a first binding component 52 secured to the harness 32 prior to gowning or after gowning which allows the operator 20 to position the first blinding component 52 beneath or on the surface of the gown as desired. Once the harness 32 is donned by the operator 20 and the operator 20 is ready to engage with the radiation protection system 10, the operator guides the first binding component 52 into a mated position with second binding component 60. Once engaged, the operator 20 is then free to move in concert with the system 10 while performing a procedure. When the operator 20 is ready to disengage from the system 10, the operator 20 simply exerts enough force to overcome the binding force between the first and second binding components 52,60 thereby resulting in the separation of the operator 20 from the radiation protection system 10. Disengagement may be accomplished by the simple wave of the operator's hand, a push movement, a twisting movement or other bodily movement, allowing the operator 20 to exit the protection system 10 with little effort and maintaining sterility. If required, the operator 20 may disengage and reengage with the protection system 10 as described above without breaking sterility or the sterile field.

**Figures 4D-4F** depict various types of configurations utilizing magnetic and mechanical forces to enable engagement and disengagement of binding components. However, it should be noted that a plurality of different configurations of components could serve the same or substantially similar functions within the scope of this invention. For example, instead of using guide blocks and guide fingers as disclosed herein, any combination of male and female shapes could serve the same or similar purposes of stabilization and limitation of motion between the two binding components when engaged, and to enhance the magnet's function of keeping the components engaged as desired. Usually, the binding shapes could mate without requiring deformation, but deformable shapes could also be employed. For example, the shapes depicted herein will mate when brought directly together in a wide variety of spatial approaches; however, different designs might require special spatial approaches to enable engagement. The shapes depicted use guide fingers that are oriented at angles that facilitate guidance of the guide blocks into proper alignment as the blocks approach the fingers from a variety of spatial positions. The angles and shapes of the guiding mechanism could be highly variable. A large bowl-type shape could be used, and shapes that are triangular, hexagonal, octagonal, or any number of angular sides could be used. Smooth shapes or sharply angulated shapes may be used on one or both binding components as desired. The shapes depicted in **Figures 4A-4C** act to guide the binding components when approach between the binding components is off-center in any spatial direction. Nonetheless, more limited guiding mechanisms may be designed to provide re-orientation of the binding components when approach between them is off-center in certain directions. The binding components shapes and properties may also be designed to perform multiple functions. For example, the guide blocks and guide fingers serve to help guide components into a mated position together when they are approaching each other off-center, but they may also act to provide mechanical stabilization once the elements are engaged, by both limiting the range of motion between the engaged components, and by increasing the magnitude and force required to disengage the parts from each other. In alternative embodiments, it would be somewhat simple to change the configurations of binding components such that these different functions would be provided by different components (i.e. the guiding elements could be separate from the stabilization elements). Additional designs and embodiments incorporating these various approaches are contemplated, yet not described in detail herein due to the vast number of possibilities.

In another alternative embodiment, the magnetic force between the strike plate 58 and magnet 66 shown in **Figures 4D** and **4E** may be controlled with electromagnetic means as known in the art. Electromagnets may be activated and deactivated by the operator via a switch means attached to either the operator's harness, the radiation protection system, to a fixed location in the suite, or externally to the system by a wired connection or wireless control system. The operator may then simply position the first binding component in relative proximity to the second binding component, initiate an electromagnetic force, resulting in the engagement of the first and second binding components. When the operator desires to exit the protection system, the operator deactivates the electromagnetic force, resulting in the disengagement of first and second binding components and exits the protection system. Alternatively, activation of the electromagnetic force could cause detachment while de-activation causes engagement of the first and second binding components, through the use of mechanical means that are over-ridden by the electromagnetic force.

Many embodiments of the disclosed invention could be integrated with a locking mechanism, such as electromagnetic or clutch locking systems, for locking and unlocking the articulations or joints of the suspension assembly, thereby coordinating the operator's entry or exit from the system with the immobilization or partial immobilization of the entire system. For example, a locking mechanism may be incorporated into the system allowing the operator to de-activate the electromagnets in the binding system for the operator to exit the system, and this action could also actuate a switch that in turn activates the locking or clutch mechanism in some or all articulations or joints of the suspension assembly, resulting in the immobilization of the radiation protection system in a "parked" mode while the operator is free from the system, thereby preventing the system from moving and/or drifting into un-sterile territory.

In alternative embodiments, the quick release mechanism may be configured so as to provide for disengagement by the operator's hand movement or other body motion. The binding system disclosed herein may be positioned in the front, rear, or side of the garment and/or frame. The harness described herein may be secured to the operator by hook and loop fasteners, magnets, snaps, buttons, straps, buckles or any suitable fastening means known in the art. In another embodiment, the harness may be sterile, donned outside the operator's gown, and operable to mate with the binding component secured to the frame or garment through the sterile drape covering the garment or frame. In a similar fashion, a sterile belt or other retention means is utilized to mount the binding means attached to the frame or garment. In this embodiment, the binding system is sterile and mates without a gown or drape between the binding components.

In another embodiment, the operator wears a non-sterile harness under her sterile gown. After gowning, a second binding component, also sterile, is attached outside the gown, using magnetic or mechanical means to bond to the harness system through the operator's gown. This outer component is equipped with binding means to bond with a binding component on the device. It could bond to the device through the drape surrounding the device, or the device could, similar to the described system above, have a second, sterile binding component that binds to the device component through the drape, and said outer component would then bond to the component attached to the operator. Thus, the binding system could be composed of multiple binding components in layers, including layers of gown or drape to provide sterility during engagement and disengagement or reengagement of the operator. This system may have advantage in allowing the mating components that are repeatedly engaged and disengaged, to oppose directly without interposed layers of sterile material. In alternative embodiments, the binding components may be located in front of, in back of, or in various locations around the circumference of the operator's person and the radiation protection system components.

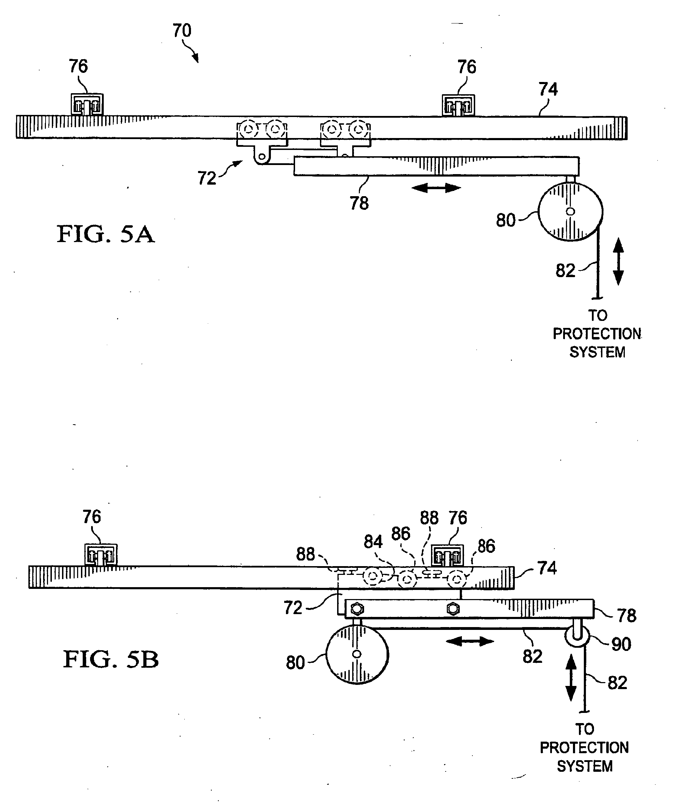

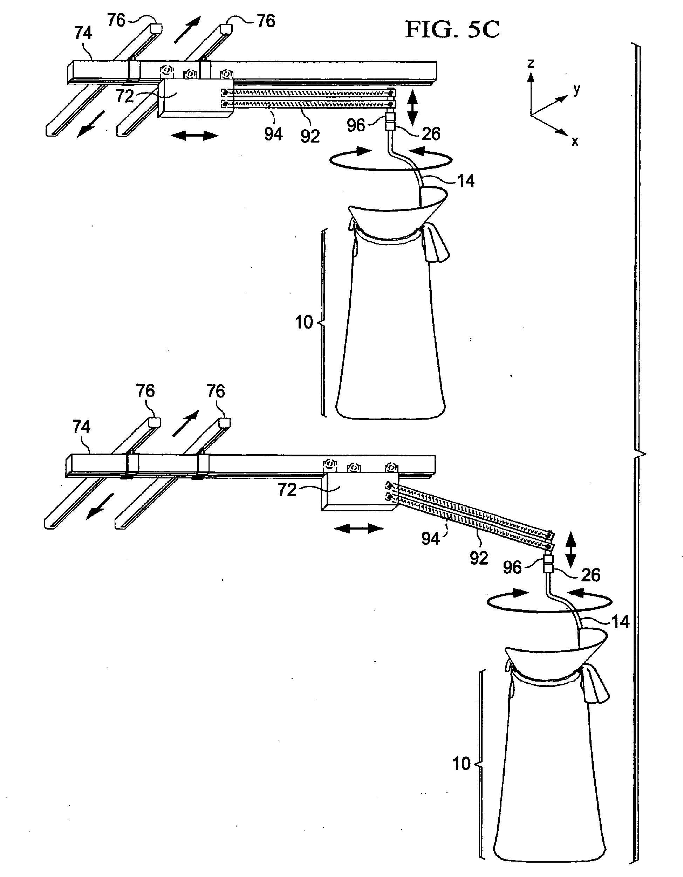

**Figures 5A-5C** depict an embodiment of an improved extension arm assembly 70 utilized in connection with the personal radiation protection system disclosed herein. As described herein, a trolley 72 is a component of the system 70 which moves linearly along the Y axis in the horizontal plane along the bridge 74, and the bridge 74 may move linearly along the X axis in the horizontal plane along rails 76 of the system, which are attached to a structural support (e.g. ceiling) and provides an attachment site for assembly 70. The assembly 70 also includes an extension arm 78 attached to trolley 72. A balancer 80 may be fixedly attached or slidably attached to the extension arm 78 along with a wire rope 82. In an alternative embodiment, an articulated extension arm may be used (shown in **Figure 5C**). The balancer acts to provide a supporting force for the protection system, by utilizing cables, springs, counterweights, pneumatic, electric or hydraulic systems as known in the art, which offer any of several degrees freedom of motion and provides substantial support for the weight of the protection system on the while providing for movement of the system in a substantially weightless manner. Extension arm assembly 70 may also include any number of articulating joints and arms as desired or required for specific applications.

Accommodating a bridge/crane setup may prove difficult in some applications, especially when rails 76 or bridge 74 cannot be positioned over an area where the suspended device may be usefully employed due to mounting limitations. In order to overcome these limitations, **Figure 5A** depicts an extension arm 78 that in the depicted embodiment is rigidly attached to the trolley 72. In one embodiment, the extension arm 78 extends beyond the patient table, extending the reach of the system 70 along a linear axis. In the embodiment depicted in **Figure 5B****,** anti-kickup wheels 84 are integrated with the trucks of trolley 72, in addition to trolley wheels 86, to counteract the additional torque on the trolley 72 and prevent "kick-up" of the trolley 72 and/or extension arm 78. Side wheels or rollers 88 may also be utilized in the trolley trucks to prevent sideways movement in the transverse plane to the lateral motion of the trolley 72.

**Figure 5B** depicts the extension arm 78 with the balancer 80 positioned so as to provide some degree of counterbalance effect to the protection system weight transmitted by wire rope 82. In this embodiment, the balancer 80 is placed underneath the trolley 72. Pulley 90 provides a rotational guide mechanism for wire rope 82 in this embodiment. This change in the position of the balancer 80 moves the center of gravity of the load suspended from the extension arm 78 closer to the trolley 72 center of gravity. This reduces the moment arm torque on the trolley 72 and the extension arm 78 and thus facilitates improved movement of the trolley 72 and reduces the structural requirements of the trolley 72 and extension arm 78, thereby decreasing the mass and overall weight of the system 70. As a result, less force is required by the operator to accelerate the system 70 by operator movement.

In another embodiment not shown, these advantageous effects can be enhanced further by positioning the balancer on an arm located on the opposite distal end of the trolley relative to the extension arm carrying the load. In these embodiments where the balancer is not directly over the supported load, the wire rope is dispensed through a system of pulleys or similar devices. Likewise in other embodiments, instead of a spring balancer, this same system could utilize other means of balancing the load such as pneumatic balancer, simple counterweights, springs, or any other means known in the art.

In an alternative embodiment not shown, the extension arm is attached to the trolley with a fixed pivot at one end of the extension arm. As a result, the extension arm may pivot freely in the horizontal plane so as to provide the operator with improved radial freedom of movement. This extra motion occurs at the expense of torque forces out of the plane of the trolley wheels, possibly requiring a wider base of support such as provided with the double girder bridge structure (as depicted in **Figure 1**) and/or another set of trolley wheels in a different plane. Alternatively, the degree of motion radially could be limited so as to keep these disadvantageous forces minimized.

In the embodiment depicted in **Figure 5C****,** an articulated extension arm 92 is attached to trolley 72 and the radiation protection system 10 with a rotatable connection between rotation joint 96 and hanger 26. Articulating extension arm 92 articulates to allow vertical motion of the radiation protection device 10 while maintaining its vertical orientation. In this embodiment, the bridge 74 and trolley 72 permit free linear motion of the radiation protection device 10 in the horizontal plane and the articulating extension arm 92 provides for substantially linear motion in the vertical plane, while also including a spring balancer mechanism 94 for counteracting the weight of the suspended radiation protection device 10. A rotational joint 96 permits rotation of the radiation protection device 10 as shown. In this way, translational motion of the device 10 is permitted anywhere in the spatial volume of the X, Y and Z axes, while rotational movement of the device 10 in the vertical planes is precluded.

In the lower figure of **Figure 5C**, the trolley 72 and extension arm 92 are shown as having moved outside of bridge rails 76 and the articulating extension arm 92 is in a lowered orientation as shown. Thus, the radiation protection device 10 has translated in the x and z spatial axes relative to the top figure shown in **Figure 5C****,** and could easily also translate in the Y axis through movement of the bridge 74 along rails 76. Other alternative embodiments may provide any number of additional degrees of freedom of motion, including rotational motion of the radiation protection device in different planes to alter the protection system orientation as needed, such as to provide pitch and roll, by using a swivel joint. Such motion may occur in the absence of translational motion of the bridge and trolley components. Any number of articulations may be provided in the extension arm system or in the hanger and/or frame to enable the desired effect.

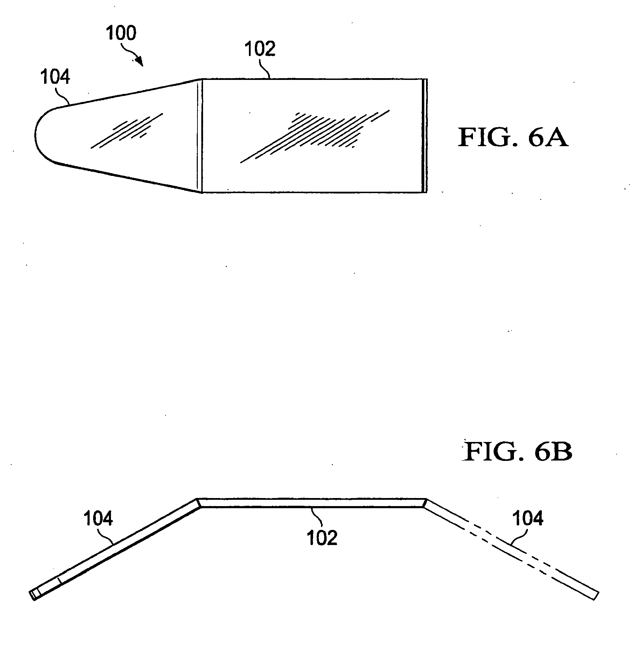

**Figures 6A-6F** depict one embodiment of an adjustable face shield system 100 which may be integrated with the personal radiation protection system disclosed herein. When performing procedures, the operator will commonly stand on one side of the patient table. Typical suites are designed asymmetrically so that one side of the patient table provides a better proximity to optimize the available lighting, shielding, controls, back table supplies, and space. The remaining space surrounding the patient table is usually available to the operator, although typically with some limitation.

For most common procedures, the operator tends to receive greater radiation exposure on the left side versus frontal exposure, while the operator's right side typically receives the least amount of scattered radiation while conducting a procedure on a patient. The geometry is such that a shield oriented to the left, front side of the operator provides optimal protection. The configuration may be changed however, when the operator moves to the opposite side of the patient table to conduct a procedure.

Various example embodiments of an adjustable face shield assembly 100 are depicted in **Figures 6A-6E****.** The shield may be manufactured from lead acrylic, lead glass, or other radio protective materials that are significantly transparent to visible light. **Figure 6A** is a front side view depicting a face shield assembly 100 with a front plate 102 and a side plate 104. The front plate 102 and side plate 104 may be provided with beveled edges so as to allow the front plate 102 and side plate 104 to be positioned together at an angle to provide a unified barrier. In one embodiment, the front plate 102 is positioned directly in front of the operator's face, while the side plate 104 is positioned at an angle to the operator's face.

**Figure 6B** depicts an alternative embodiment of the shield wherein the side plate 104 may include beveled edges on opposite sides, so that it may utilized on either the right or left side of the front plate 102 depending on the operator's position. This embodiment allows the operator to remove a side plate 104 from the one side, and affix it relative to the opposite side of the front plate 102, by simply inverting the side plate 104 and securing it to the frame 14. This precludes the necessity of having a "left" and "right" side plate, as one side plate 104 may be utilized as a "left" or "right" side plate at the behest of the operator and the orientation of the system to the patient table.

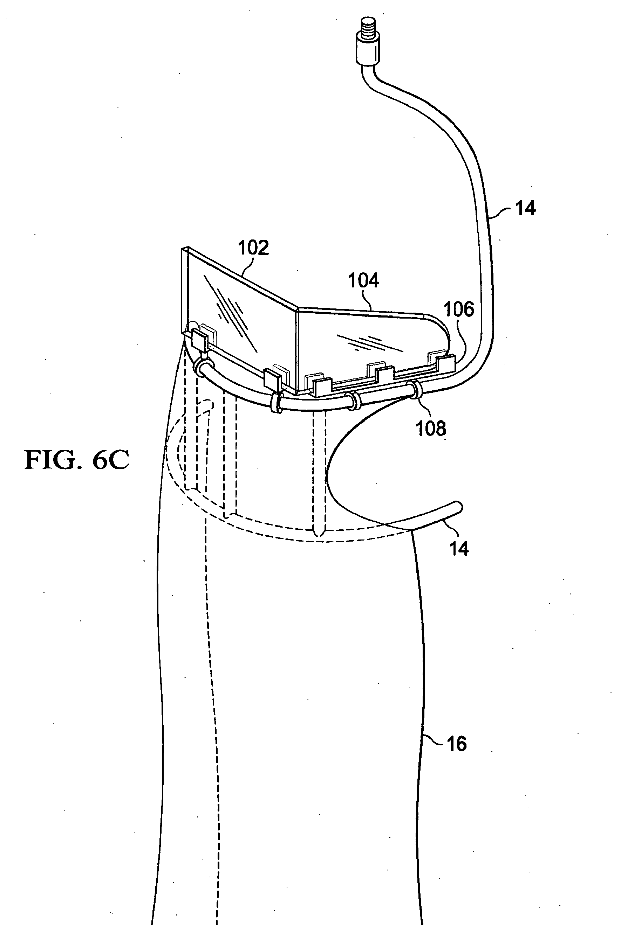

As shown in **Figure 6C****,** shield plates 102,104 are shown detachably secured to the frame 14 via shield retention bar 106. Shield plates 102,104 may be secured to the frame 14 using friction type fasteners, such as rubber grommets or other known fasteners that provide a sufficient grip between the shield retention bar 106 and shield plates 102,104. Shield retention bar 106 may be detachably secured to frame 14 via clamps 108. Clamps 108 may include clamping means that are well known in the art, such has screw clamps, hose clamps, or other reliable clamp fittings. In alternative embodiments, the shield retention bar 106 may be integrated directly with the frame 14 or welded thereto, or clamps 108 may also be utilized to attach garment 16 to frame 14. The side plate 104 may be detachably secured to either the left or right of the front plate 102, thereby allowing the operator to adjust the shield configuration to her preference. Multiple shield plates may be designed or shaped to provide protection to the operator in a variety of operational environments or the preferences of the individual operator.

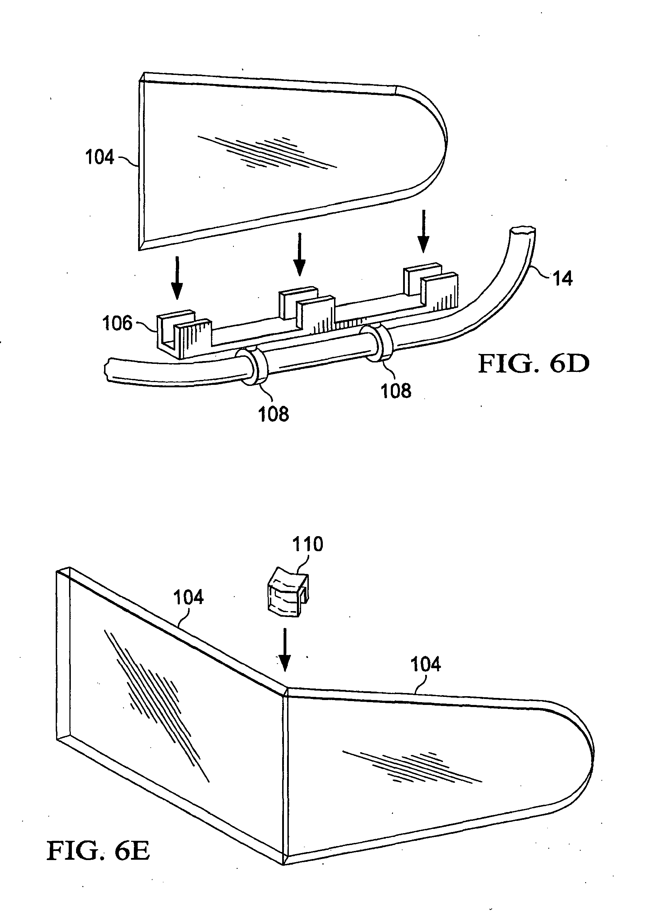

**Figure 6D** depicts another embodiment of the adjustable shield disclosed herein. A shield retention bar 106 is shown detachably secured to the frame 14. Retention bar 106 is secured to the frame 14 via clamps 108. Retention bar 106 may be fabricated out of metal, rubber, polymers, ceramics or any material capable of supporting and retaining the face shield plates, in the depicted embodiment side plate 104. Retention bar 106 provides a detachable connection for securing the shield plates 102,104 to the frame 14. In the depicted embodiment, the operator simply slides the shield plate 104 into the retention bar 106 where the friction or retention force in bar 106 retains the shield plate 104 in the desired position. The operator may move the shield plate 104 along the retention bar 106 as desired for placement and protection.

A shield connector 110 is shown in **Figure 6E** wherein the top portion of the front shield 102 and side shield 104 may be further secured relative to one another by means of a connector 110. Connector 110 may be constructed of rubber, nylon or other suitable material capable of maintaining a friction connection that may be affixed at the top or side edges of the shield plates 102,104 where they are in close proximity to keep the shield plates 102,104 in position relative to one another. Connector 110 may also be used if side plate 104 was inverted and moved to the opposite side.

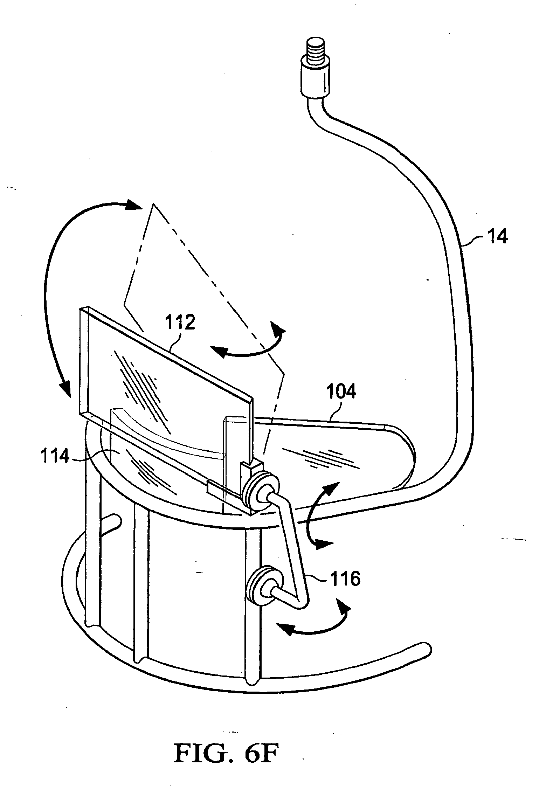

**Figure 6F** is a depiction of an alternative embodiment where the shield 112 or a portion of a modular shield, is secured to the frame 14 via arm 116. As depicted, a side shield 104 is affixed to the frame 14. Similarly, a small front shield 114 with a low height is fixed to the front of the frame 14 and is held in a substantially fixed position. Shield 112 is attached to the frame 14 by an arm 116 that in some embodiments has flexible joints, pivots, or hinges at each end, where it attaches to the frame 14 and where it attaches to the shield 112. The flexible joints, which comprise a ball-in-socket joint in the embodiment shown, provide many degrees of freedom of motion. The front shield 112 can be manipulated and moved by the operator to a desired position in all spatial planes. For example, the front shield 112 may be positioned outside of the operator's line of sight when performing a procedure where vision is critical and shielding by the front shield 112 is not required. Arm 112 allows the operator to adjust the attached shield 112 or additional shield plates (not shown) to the optimal orientation for protection and/or glare reduction.

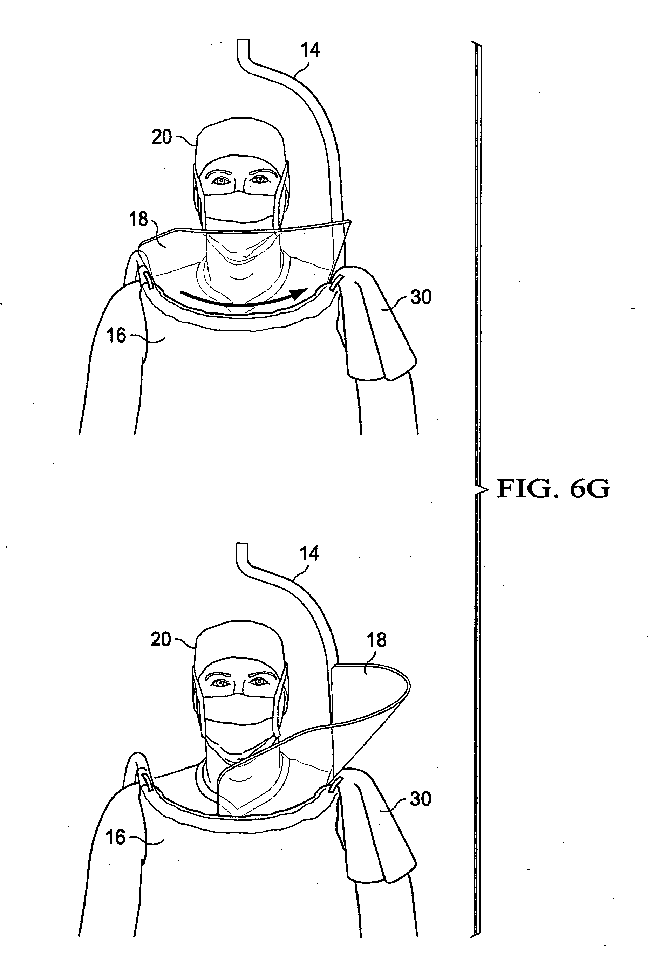

The shield system described herein may be secured to the frame by friction connection means, rigid means, by an extension arm assembly, or by other means as is known in the art. In one embodiment depicted in **Figure 6G****,** a single piece curved shield 18 may be slidably connected to frame 14 along a sliding track integrated with frame 14. The operator 20 may simply move the shield 18 along the curvature of the frame 14 with her hand, while shield 18 remains in position with the frame 14. Such capability makes it simple for the operator 20 to move the shield 18 position, to reduce glare from the shield 18 and/or to improve radiation protection to the operator 20 due to angle of scattered radiation during a particular procedure. This embodiment allows the operator 20 to quickly and easily move the orientation of the shield 18 without the need to detach or physically detach and reattach the shield 18 from the frame 14. Likewise, the orientation and position of the shield 18 may benefit from occasional repositioning during an actual operation with a patient, while allowing the operator 20 to move and/or manipulate shield 18 while remaining sterile.

In alternative embodiments, any number of shield plates may be attached to the frame at various locations on the frame as desired by the operator or as required by the procedure. Alternate embodiments could involve more than 2 flexible joints per arm. For example, an articulated arm with a joint in its middle section could be used for increased freedom of motion. Alternate embodiments could include any number of movable or stationary shield components slidably connected to the frame as discussed above. Also, any type of joint is possible, with any different number and combination of degrees of freedom of motion. A flexible arm system, or "snake" as is sometimes used with common table lamps, could be utilized. Other embodiments may not utilize fixed shield components, and all components could be movable on flexible joints or arms. Other embodiments may utilize one large, curved or angled shield, or flat shield, on a flexible attachment that can be moved about by the operator to provide optimum shielding and glare reduction. In another embodiment, a handle mechanism may be fashioned for attachment to a portion of the shield or arm to allow movement of the shield by sterile hands of operator without contamination. Alternatively, a sterile cover or drape may cover a portion of the moving system to allow such manipulation.

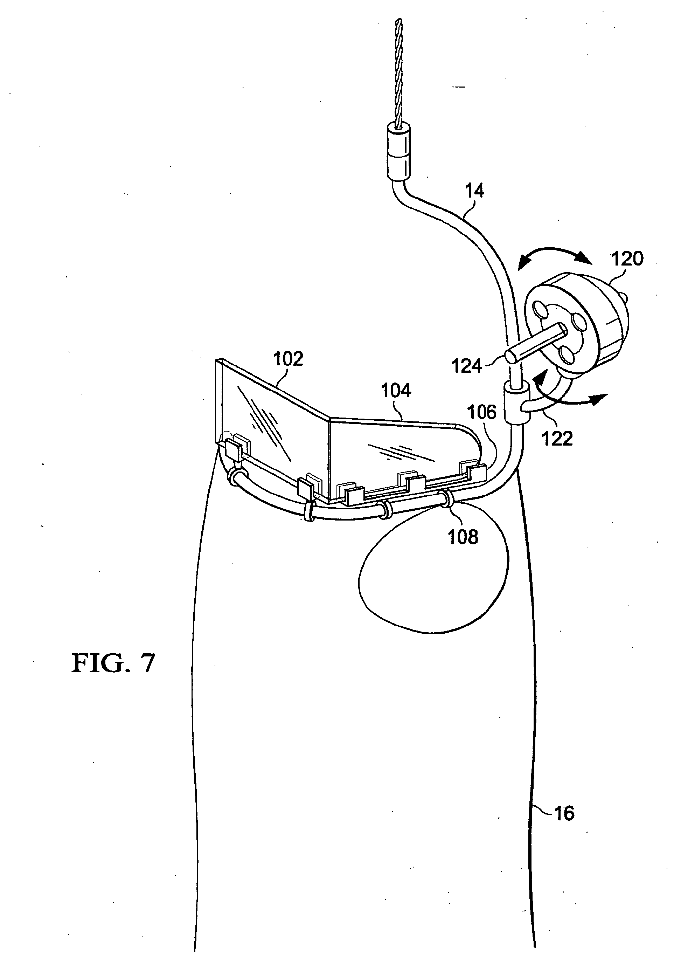

**Figure 7** depicts an embodiment of the personal radiation protection system disclosed herein with a lighting apparatus attached therewith. During the conduct of a procedure, lamps or other lighting means are used to facilitate the conduct of the procedure. As can be imagined, sufficient lighting must exist in the suite so that the operator may skillfully and safely conduct a procedure. Ideally, the lighting mechanism is powerful, capable of being manipulated for optimum direction, and have a broad reflective base to provide a wide beam of light with minimal shadowing. In some procedures, the operator conducts the procedure by viewing a monitor which projects images received from camera feeds.

In some operational suites where space is confined and at a premium, it would be preferable to utilize a smaller lighting source which may be attached to the system disclosed herein. As shown in **Figure 7****,** one embodiment of the invention disclosed herein includes a lamp or light source 120 connected with the frame 14 via arm 122. A manipulation handle 124 may be included which allows the operator to adjust the direction of the light source 120 with a simple hand movement, while allowing the operator to maintain optimum light direction on the subject. Alternatively, lamp 120 could be connected directly to the frame 14, garment 16, face shield 102, side shield 104 or any portion of the system generally described herein.

Light source 120 may also be integrated with the system disclosed herein so that its weight may be used as a counterweight, providing an additional mechanical advantage to the entire radiation protection system. Light source 120 may be positioned in a variety of positions relative to the operator as desired. Light source 120 may be powered by typical electrical means integrated within the system as is known in the art. In one embodiment, light source 120 may be battery powered and therefore wiring and electrification of the system, or a portion of the system, would not be required. Batteries to power the light source 120 may be rechargeable, so that charging is performed during periods when the system may not be in use.

In addition to lighting systems, other alternative embodiments may include environmental controls such as fans or air blower/heater systems integrated with personal radiation system disclosed herein. Such environmental control systems may be affixed to the suspension assembly, frame, garment or any other component of the system disclosed herein. **Figure 7** also serves to depict a frame embodiment wherein the frontal frame element sweeps around the lower head, neck, or upper torso region of the operator, but does not contain a lower frame below it. Instead, the garment hangs or is supported entirely from the upper frame element. In this embodiment, the left arm protrudes through an arm hole.

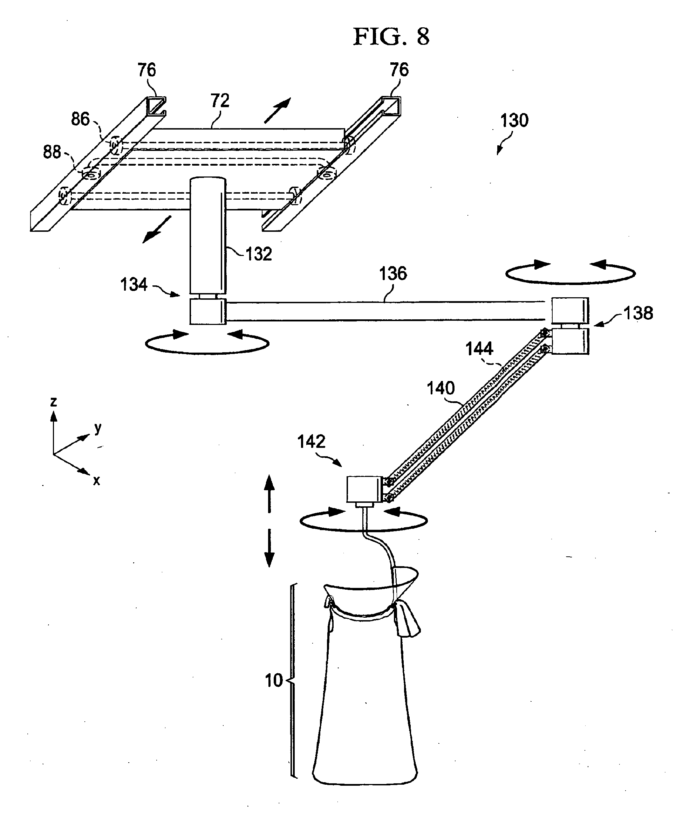

**Figure 8** depicts an alternative suspension system 130 which may be utilized with the personal radiation protection system disclosed herein. Suspension system 130 includes a post 132 attached to a moveable crane system including a bridge/trolley 72 that can roll linearly along the Y axis on wheels 86 and side wheels 88 within ceiling rails 76 as shown. In alternative embodiments, the suspension system 130 may simply be attached to a fixed point such as a wall or ceiling, for example by attachment of post 132 directly to the ceiling. In the embodiment shown in **Figure 8****,** the bridge/trolley 72 may be locked in position relative to the ceiling rails 76 when only suspension arm motion is desired. A first arm 136 is connected to post 132 such that it is oriented horizontally and may rotate in the horizontal plane, parallel to the plane of the floor about joint 134. A second extension arm 140 is connected to the first arm 136 via joint 138 and is operable to rotate in the horizontal plane about second joint 138 which allows rotation of extension arm 140 in the vertical plane. Extension arm 140 is operable to rotate in the vertical direction as well and operates to articulate in the vertical plane with spring tensioners 144 assisting in supporting radiation protection system 10 connected therewith via joint 142. Joint 142 allows rotation of the radiation protection system 10, for example a "spinning" or "twisting" motion of the radiation protection device 10, as might occur when the operator moves, turns or pivots. Joint 142 may also be configured to allow rotation in the vertical plane of the of the second arm 140 relative to frame 14. However, the second arm 140, joint 138, and joint 142 may be designed in certain embodiments to prevent rotation of the radiation protection device 10 in the vertical planes of space, thus preventing pitch or roll of the system 10. As a result, the radiation protection system 10 is operable to translate in all X, Y and Z spatial directions due to the rotational capability of joints 134,138,142, and to "twist" or experience yaw, without tilting, or experiencing pitch or roll. This freedom of movement may also be accomplished by the use of linkages in reaction arms, torque arms, and manipulator arms, commonly known in the art to provide such function and which are frequently used for suspended lamps, tools and other objects. Pitch and roll motion of the radiation protection device 10 can easily be allowed by the addition of joints in the arms 136, 140, or in the radiation protection system 10, as may be imagined in alternative embodiments. In alternative embodiments, the linkages at the ends of the first arm 136 may also integrate balancing mechanisms, springs and/or cables that offset the weight of the radiation protection system 10 and maintain the system 10 in the orientation that is set by the operator. Altogether, the disclosed system 130 permits positioning of the radiation protection system anywhere in the X, Y and Z space defined by the geometries of the arms and their motions.

In another embodiment not shown, a third arm may be removably secured directly to the frame supporting the radiation protection system with either a fixed connection or flexible connection as is known in the art. In other embodiments, additional joints will connect these structures to provide various desired degrees of motion therein. In still other embodiments, a plurality of joints and arms may be configured as desired. The joints may provide any combination of degrees of freedom as to movement in the X, Y and Z planes and various balancing means may be utilized with the system. Examples of such variations may include arms with multiple joints each allowing radial motion in the horizontal plane, with numerous short arms or "links" as in a bicycle chain restricted to movement in a "sidewinding" motion. The arms would thus all be oriented horizontally, and permit great freedom of motion in the horizontal plane with less limitation than a single, double or triple arm system. The radiation protection system could be suspended by a spring balancer with wire ropes or other means such as a balancing arm at the terminus. Alternatively, a manipulation system is envisaged with multiple arms, links, or segments with balancing mechanisms built into each linkage, or some of the linkages, along with several degrees of freedom in the linkages to allow vertical motion, so that the end of the arm system can move in the vertical as well as horizontal planes, and a spring balancer with cable is not necessary to keep the load balanced. Such a system may provide more flexibility and an increased range of motion.

In still another embodiment of the disclosed invention, it may prove desirable to have the ability to secure the radiation protection system to a fixed point or "park" or store the system while it is not in use. An internal lock system may be provided which places all or some of the joints in the system described in **Figure 8** in a locked position by the provision of frictional force or an actual mechanical locking mechanism. This force may be applied via clutches, friction plates, mechanical locks, electromagnetic force or by other means as known in the art at joints 134, 138, 142. Such a parking mechanism may be integrated with the binding system disclosed previously such that activation of the park mechanism is nearly simultaneous with disengagement of operator from system, and reengagement by the operator de-activates the park mechanism for return of free motion.

For example and with reference to **Figure 8****,** electromagnets may be positioned at any or all of the joints 139, 138, 142 connecting post 132 and arms 136,140, as well as being integrated in appropriate positions within the bridge/trolley 72. The entire system is then placed into an appropriate storage position and a sufficient electrical current may then be applied causing the electromagnets located at or near joints 134,138,142 and/or within the bridge/trolley 72 act to form a sufficient binding force preventing the movement of each desired joint or component. In an alternative embodiment, a transient force may be initiated to activate a mechanical lock for each respective joint 134,138,142.

In another embodiment not shown, the operator may desire to place the personal radiation protection device in a stationary, fixed position while performing a procedure. The operator may simply park the device in a fixed position by actuating the electromagnetic system described above thereby fixing the personal radiation protection system in a fixed position. When the operator desires to move the device, she may simply disengage the electromagnetic force by activation/deactivation of a switch or other controller means, allowing the personal radiation protection system to freely move at the operator's discretion. Various components of the system may be fitted with such locking devices, so that only certain joints or components of the entire system may be placed in a fixed or unfixed position as desired.

Other systems may be utilized for keeping the device immobilized. A magnetic system may be used, whereby a magnet is present on the patient table, a portable stand, or the operator's back table. This magnet could engage a metallic strike plate affixed to the radiation protection device when it is placed in proximity, and would function even through the sterile drapes located on the various devices and equipment. The magnet could be sterile, or covered in a sterile cover or drape. Alternatively, the positions of the magnet and strike plate could be reversed, or magnets could be used in both positions. Other embodiments could use mechanical fastening means other than magnets, such as hook and loop fasteners, mechanical hooks, or any other systems widely known in the art. These could be covered with sterile drapes or provided in a sterile condition for use.

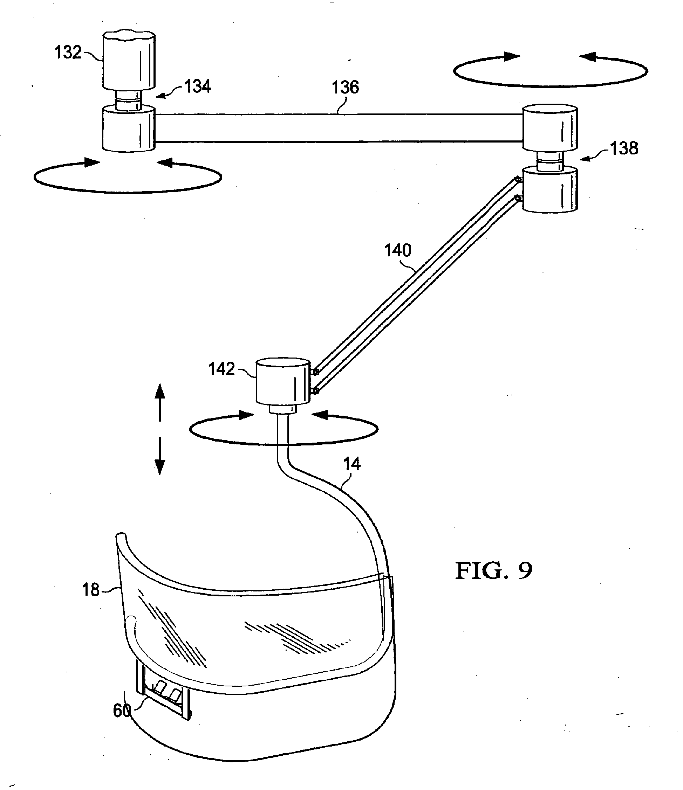

**Figure 9** depicts an alternative embodiment of the invention described herein, where the radiation protection garment may be omitted entirely from the system, which would be modified to provide an optimum platform for the suspension of the face and upper torso shield only. **Figure 9** depicts an alternative embodiment where frame 14 is supported by a suspension system including post 132, arm 136, and articulating extension arm 140 in a manner that maintains orientation of the frame 14 and shield 18 relative to the operator. Joints 134,138,142 provide rotational connections as described herein allowing the system to move in the desired planes of motion. When using such a device, protection of the operator's torso and extremities would be provided by some other protection means such as a standard apron worn by the operator or other shield device. This might be desired by an operator who does not mind wearing and supporting the conventional heavy lead apron, or who prefers not to have the previously described suspended garment system for any reason such as any notion of encumbrance. This suspended system would be much lighter without the garment and could be supported by lighter and less expensive suspension apparatus. The depicted embodiment includes a transparent shield 18 that curves around the sides and front of the operator's' head and upper torso, permitting free operator arm motion. In one embodiment, the shield 18 may be connected to a frame 14 that sweeps under the shield 18 as shown in **Figure 9****,** or alternatively frame 14 could support the shield 18 along the top margin of the shield 18, or the frame 14 could attach to the left or right side of the shield 18, or to another frame component that surrounds the shield 18 like a window frame. Shield 18 may be rigidly, slidably attached or movably attached to frame 14 as described herein.

In the embodiment shown in **Figure 9****,** the frame 14 may include a binding component, such as binding component 54 or 60, for use with the magnetic binding system disclosed herein. In other embodiments, frame 14 may have one or more articulations within it to provide motion in any direction, or to change the orientation of the shield 18. Frame 14 may be structured to accept a sterile drape to maintain sterility of the operator when in proximity to her, and when the operator must grasp frame 14 to move it. Frame 14 may also include a handle to assist in moving or manipulating frame 14. In other alternative embodiments, the system disclosed herein may employ some or all of the possible functions, accessories and suspension means described in this document.

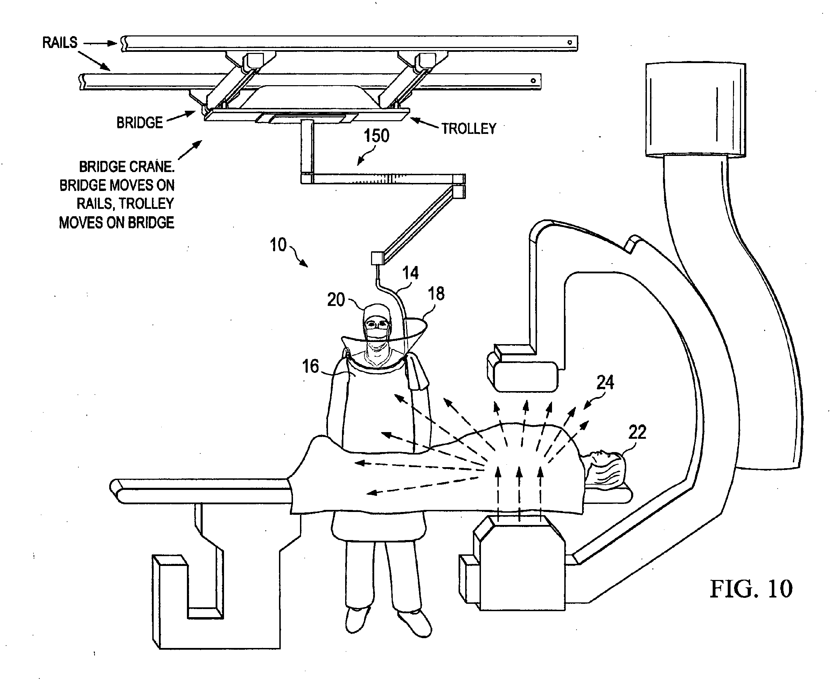

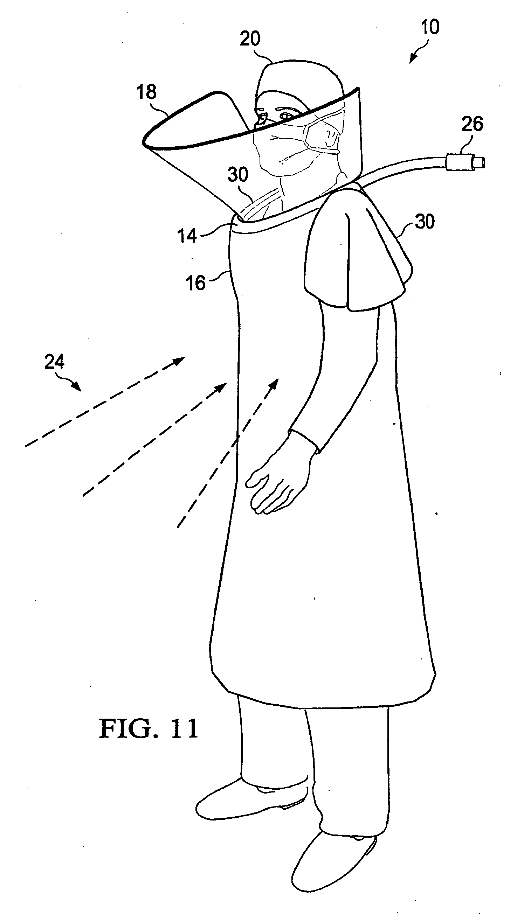

**FIGURE 10** is a perspective view of one embodiment of a suspended personal radiation protection system 10, which is shown in greater detail in **FIGURE 11** where a rigid, substantially vertical suspension component of a ceiling-mounted articulating arm suspension system 150 is used to directly attach to a radiation protection garment 16 in accordance with the present invention. System 10 may also include an operator 20, a patient 22, radiation rays 24, a suspension component 150, and frame 14 for supporting a radiation protection garment 16. The system 10 may also include a face shield 18, and an arm hole sleeve cover (not shown). Each of these components is discussed in further detail herein.

In general, and as illustrated in **FIGURE 11****,** the garment 16 is suspended from a frame 14 which is in turn supported by a given suspension component. In one embodiment, hanger 26 is attached to frame 14 (or "garment frame"), which is the skeleton that contours around the shoulders, chest and torso of the operator 20. Frame 14 supports garment 16 and face shield 18, along with other devices such as an instrument tray, environmental control (e.g. a fan, a heater, an air conditioning device) and/or lighting apparatus. Frame 14 may be integratedrigidly or with articulation--with hanger 26, or may be attached directly to the suspension component as required by operational mandate. In one embodiment, hanger 26 is a rigid rod that connects frame 14 to the desired suspension component, such as a reaction arm, balancing arm, a manipulating arm ("manipulator"), an articulating arm, any number of wire ropes, or any other suspension component described or envisioned herein. Attaching frame 14 to a suspension system over the operator's head, and roughly over the center of gravity, can have advantages for facilitation of balance and proper orientation of the device. Alternatively, attaching frame 14 in any other location, such as behind (as depicted in **FIGURE 11****)** or to the side of the operator 20, can offer advantage with regard to positioning of the suspension system in an alternate location, other than overhead the operator 20, for better function in some environments.

**FIGURE 11** further illustrates the system architecture by a perspective view of a personal radiation protection system 10. An operator 20 may position herself in relative proximity to the garment 16 and frame 14 such that the operator 20 is not supporting the weight of the garment 16. In this sense, she is liberated from the typical and problematic weight constraint. While using radiation 24 during a procedure or task, the operator 20 can freely move in the X, Y and Z spatial planes such that the garment 16 and face shield 18 are substantially weightless. Garment frame 14, or when coupled with hanger 26, can lead upward from any portion of the garment system 10 to attach to an overhead suspension system such as a bridge system or reaction arm. Alternatively, frame 14, or when coupled with hanger 26, can lead to the suspension system in a non-overhead location by passing rearward, and/or to the side of the operator 20, as shown. Some embodiments of the invention depicted may also utilize a gimbal, end effector, or other type of articulation within the frame 14 or hanger 26, close to the operator 20 or near the center of gravity of the system 10 to facilitate certain movements, as described later herein. The rearward or sideward frame component 14 may arise at other levels of the operator 20 other than the shoulder, such as closer to the waist, or midway between shoulder and waist, or possibly lower.

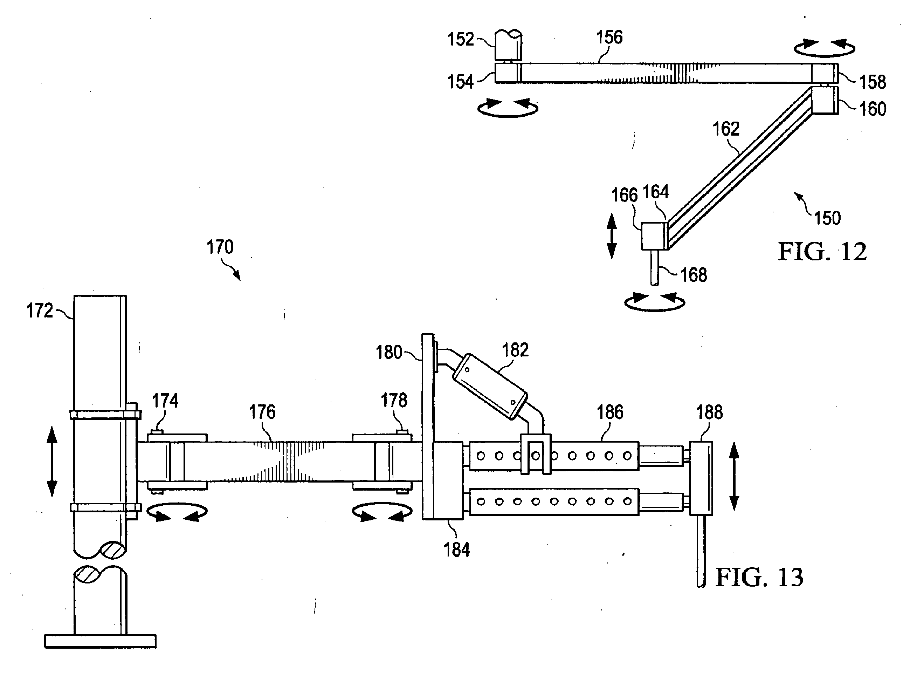

**FIGURE 12** is an elevated side view of the manipulator-arm-type, or reaction-arm-type, garment suspension system 150 seen in **FIGURE 10****,** in accordance with the present invention. A manipulator arm suspension assembly 150 allows the operator to move freely in the X, Y, and Z planes while maintaining proximity to and protection by the protection system. Suspension system 150 includes a post 152, which, in this embodiment, is attached to a moveable system consisting of ceiling rails and a trolley on wheels as depicted in **FIGURE 10****.** Post 152 alternatively could be attached to a fixed point, such as a wall or ceiling. A first arm 156 is attached to the post 152 such that the first arm 156 may rotate about a rotating first joint 154 in a planar fashion. A second rotatable arm 162 is attached to the first arm 156, and is also operable to rotate about a rotating second joint 158 in the X, Y plane and to rotate about a rotating third joint 160 in the Y, Z plane. The second and third joints 158, 160 may alternatively be combined into a single ball-and-socket (or "ball-and-cup") joint to provide rotation in the X, Y, and Z space. A third arm 168 is operable to rotate about a rotating fourth joint 164 and a rotating fifth joint 166 in various planes relative to the second arm 162. Similarly, the fourth and fifth joints 164, 166 can be combined into a ball-and-socket joint. The joints connecting each of the arms described above are well known in the art and may consist of friction joints, torque joints or substantially frictionless connection joints using well-known means such as bearings or bushings.

In this particular embodiment, arm 162 contains a linkage system (not depicted) that maintains the orientation of third arm 168 in the vertical axis, perpendicular to the first arm 156. As fifth joint 166 allows rotation in only 1 vertical plane, instead of infinite vertical planes as with wire rope, the remainder of the arm 168 will more responsively follow the operator as he or she moves, starting and stopping in concert with the operator. In contrast, with wire rope suspension systems, the rope will first angle before pulling the arm, which may then give some undesirable backlash effect as it passes over the operator in a delayed manner, and comes to rest after the operator.

The depicted device may also contain (not shown) an internal spring and cable mechanism that acts upon joint 160 between second arm 162 to first arm 156 to maintain an upward force of the suspended protection system approximately equal to its weight. It may have friction built into the joints so that there is some difficulty moving them, and then when the operator releases, the systems remains. In an alternative embodiment, the joints could be substantially free of friction to allow more fluid motion while working.

**FIGURE 13** is an elevated side view of an alternative suspension component (which can be used in place of the articulating/manipulator arm system 150 depicted in **Figure 10****)** comprising another embodiment of a reaction arm suspension assembly 170, in accordance with the present invention. Arm suspension assembly 170 is a type of articulating arm that can provide a predetermined amount of upward force to counteract the force of gravity that acts upon the desired tool or load to be attached at the end of the arm suspension assembly 170. A reaction arm (or torque arm) with parallelogram construction (i.e., dual, parallel hinged arms acting as one unit), such as that depicted, can allow translation in the X, Y, and Z space of the suspended load, while maintaining the load's pitch orientation. Parallelogram construction maintains effector pitch orientation even in the face of opposing torque forces that would otherwise result in rotation of the object (i.e. undesirably changing its orientation in the YZ plane).

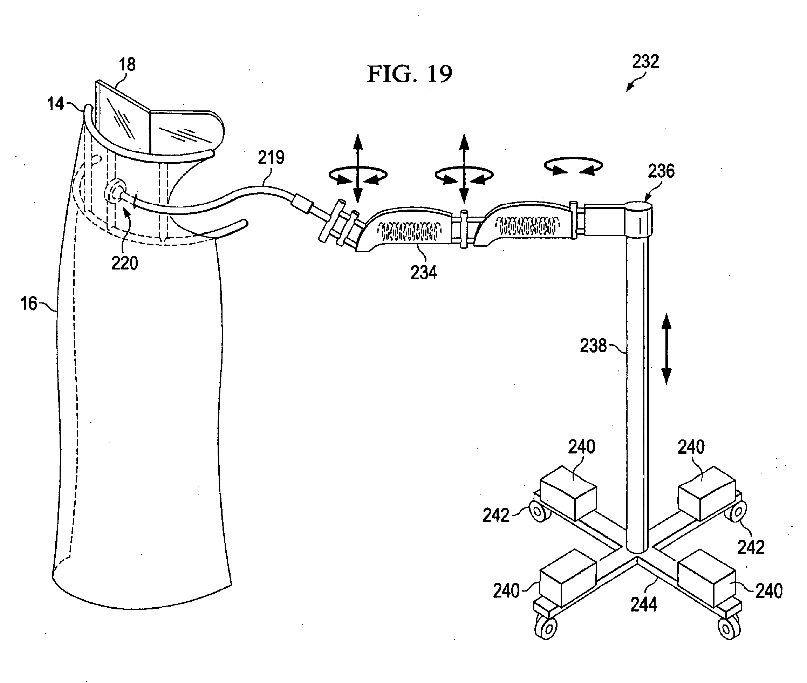

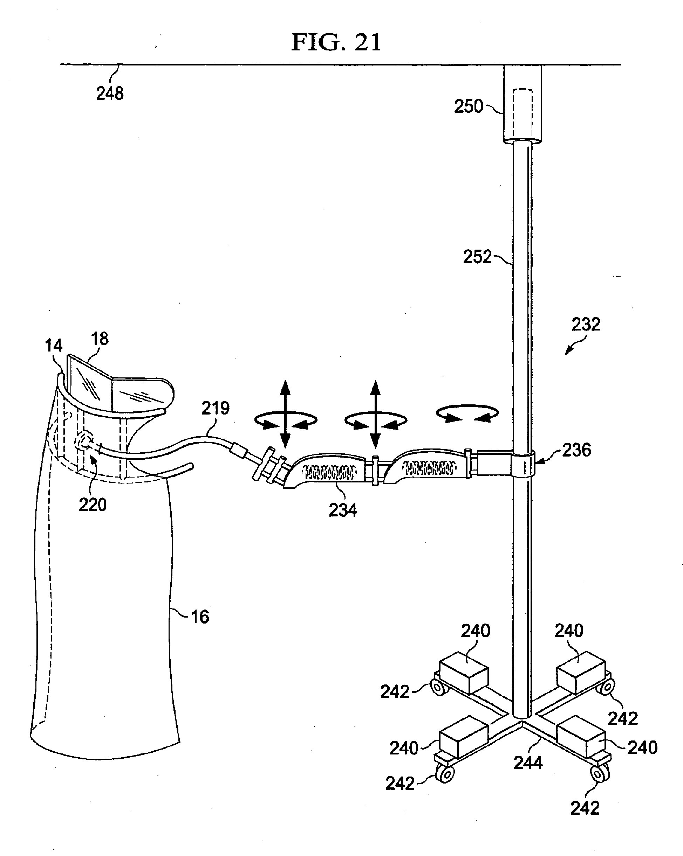

Balancing arm assembly 170 shown in **FIGURE 13** is a heavy duty articulating arm assembly with two boom or arm sections 176, 186 extending serially, via rotating joints 174, 178, 184, 188, from a mounting post 172. The mounting post 172 can extend upward to attach to the ceiling or a ceiling-mounted structure, such as the trolley and bridge crane assembly shown in **FIGURE 10****.** Alternatively, the mounting post 172 can extend downward to attach to the floor or a floor-based stand, such as any one of the examples shown in **FIGURES 19****,** **20****,** **23****,** and **24****.** The mounting post 172 can even extend from floor to ceiling, as can be seen in **FIGURE 21****.**

Returning to balancing arm assembly 170 of **FIGURE 13****,** the first boom (or arm) section 176 rotates in a horizontal plane around mounting post 172 via a first joint 174. Second boom 186 also can rotate in a horizontal plane about a second joint 178 located at the end of first arm 176 opposite mounting post 172. Second boom 186 is also capable of rotating (tilting, swinging, or swiveling) in a vertical plane about a third joint 184. If it is desirable for the load-handling end of second boom 186 to maintain its pitch (i.e. forward/backward angle relative to vertical) regardless of the pitch/tilt of second boom 186, second boom 186 may comprise parallelogram construction (i.e. a pair of parallel arms and end joints). Hydraulic cylinder 182 can be used to provide a weight-counterbalancing force to the end of second boom 186. One end of hydraulic cylinder 182 attaches to a leverage extension 180, which may extend upward or downward from the end of boom 186 next to third joint 184, while rotating joint 188 provides a rotating support for system 10. The other end of hydraulic cylinder 186 attaches to some point along second boom 186 to provide a torque about third joint 184 to oppose the torque caused by gravity on the load-bearing end of second boom 186. A zero-gravity, load-balancing effect is thereby provided for the operator. In other embodiments, many other types of balancing mechanisms or linkages are possible, including the use of cables, springs, and reels in various linkage systems. Other degrees of freedom of motion in various joints can be added or subtracted to provide the necessary freedoms and limitations to fit the working situation.

**FIGURE .14** is an elevated side view of another alternative suspension component comprising a manipulator arm assembly 190 that is holonomic (i.e., having six degrees of freedom) or perhaps even redundant (i.e., having seven or more degrees of freedom), where the manipulator arm 190 has a wrist 204 having at least three degrees of freedom for supportive attachment to the frame 14, in accordance with the present invention. A redundant or at least holonomic manipulator 190 is particularly advantageous because its range of motion and flexibility of orientation approximate those of the human arm.

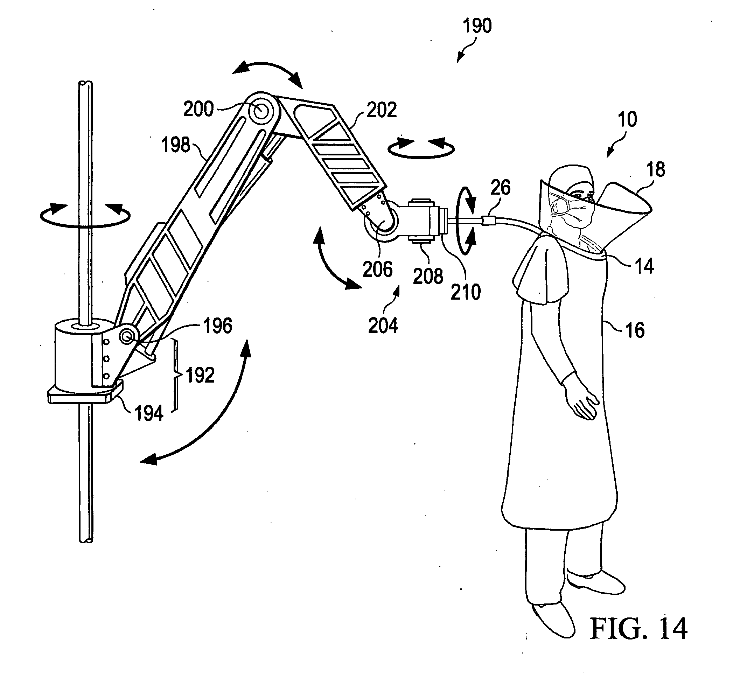

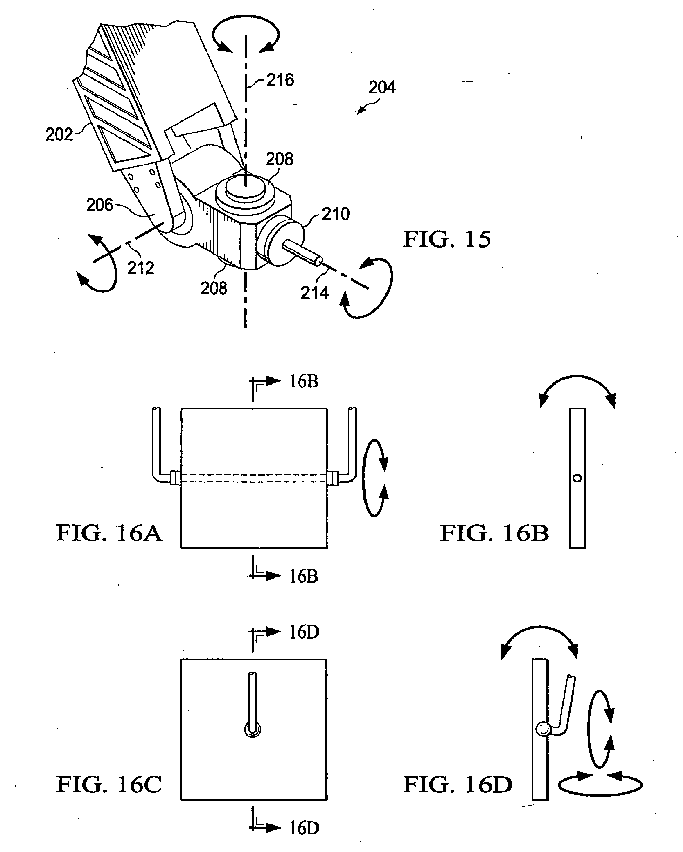

A human arm is considered to have seven degrees of freedom: a shoulder gives pitch, yaw, and roll; an elbow provides pitch; and a wrist allows for pitch, yaw, and roll. While three degrees of freedom enable positioning in three-dimensional space, additional degrees of freedom are needed to adjust the orientation (pitch, yaw, and roll) of the end effector (or hand). Three degrees of freedom in the manipulator arm shown in **FIGURE 14** enable the positioning of the end effector at any location in space (defined, for example, by X,Y,Z coordinates). The shoulder portion 192, which comprises a rotatable base 194 and a shoulder pitch joint 196, provides yaw and pitch motion to the manipulator arm assembly 190. The rotatable base 194 enables rotation or yaw about an upright post for securing the manipulator arm assembly 190 to a stable point above and/or below the manipulator arm assembly 190 (such as to an overhead bridge and trolley as seen in **FIGURE 10****,** or to a floor stand such as that shown in FIGURES 19, 20, 23, or 24). The shoulder pitch joint 196 enables an upper arm 198 of the manipulator arm assembly 190 to tilt or pitch upward or downward. A lower arm (or forearm) 202 connects to the end of upper arm 198 via an elbow joint 200, which also allows for upward and downward pitch. At the opposite end of lower arm 202 is a robot wrist portion 204, which is rotatable about a wrist pitch joint 206. The robot wrist (or mechanical wrist) 204, which is shown in more detail in **FIGURE 15****,** provides an additional three degrees of freedom for orienting the end effector to a desired pitch, yaw, and roll.

To help provide a zero-gravity-like environment for the shield 18 and/or garment 16 at the end of the manipulator arm 190, a hydraulic cylinder can span between the upper arm 198 and lower arm 202 to apply a counterbalancing torque about the elbow joint 200. A hydraulic cylinder can similarly span between the lower arm 202 and the wrist 204 to provide counterbalancing torque about the wrist pitch joint 206.

In the depicted embodiment, frame 14 can attach to the wrist 204 of the manipulator arm 190 (via a hanger 26, if desired). To facilitate the ease of manipulating and orienting the shield/garment 18, 16, the hanger 26 is more preferably located as close as reasonably possible to garment 16 and shield 18 center of gravity. This concept will be discussed in further detail in the description of **FIGURES 16-18****.** If desired, a seventh degree of freedom can be provided to the manipulator arm system by using a manipulator hand connected to the end of the wrist 204 that is capable of surge (i.e. extending forward and retracting backward). The hanger 26 and/or frame 14 may be made considerably shorter, allowing the robot wrist to be much closer to the operator, or closer to the center of gravity of the radiation protection system. This may provide more facile fine movements of the operator during pitch, roll, or yaw of his body. This may also be provided by altering the shape of the frame 14 or hanger 26. Alternatively, additional joints of various configurations and functions may be placed within hanger 26 and/or frame 14 to provide additional degrees of freedom and means for connection to the manipulator arm assembly 190.

**FIGURE 15** is a perspective view of the wrist 204 of the manipulator arm assembly 190 seen in **FIGURE 14****,** as well as the three perpendicular axes of rotation 212, 214, 216 for its three rotating joints 206, 208, 210, in accordance with the present invention. The wrist portion 204 of the manipulator arm assembly 190 provides three degrees of freedom and approximates the flexibility of a ball joint (and the flexibility of the human wrist) by combining three perpendicular joints in a relatively compact space. The wrist pitch joint 206 connects the lower arm 202 to one end of the wrist 204 and has a horizontal axis, which enables the wrist 204 to tilt upward and downward, thus providing pitch. A wrist yaw joint 208 is located in the middle of the wrist 204 and has an axis orthogonal or perpendicular to that of the wrist pitch joint 206, thus providing yaw. At the wrist end opposite the wrist pitch joint 206 is a wrist roll joint 210. The wrist roll joint 210 comprises a rotatable base for providing roll. The wrist roll joint's rotational axis 214 is orthogonal or perpendicular to the axes of rotation 212, 216 of both the wrist yaw joint 208 and the wrist pitch joint 206.

The closer in proximity that each of the wrist joints can be to one another, in the closer the wrist will be to behaving similarly to a spherical wrist. A spherical wrist is where the three axes of rotation actually intersect. Note that in the particular embodiment shown in **FIGURE 15****,** although the wrist yaw axis 216 and the wrist roll axis 214 do intersect, the depicted wrist 204 is non-spherical because the wrist pitch axis 212 and the wrist yaw axis 216 do not intersect. Spherical wrists (compared to non-spherical wrists) can be more compact and can reduce the degree of manipulator arm movement necessary to re-orient the shield/garment while maintaining the shield/garment's current spatial position. Non-spherical wrists, however, can be mechanically simpler and more robust.

In order to provide proper balance of the radiation protection device and orientation in space, some of the joints permitting some of the degrees of freedom of the robot arm and wrist may include balancing systems to counteract the effects of gravity on the arm components and the radiation protection device, to render it substantially weightless. Such counterbalancing mechanisms will usually be used to counteract motion in the Z axis (vertically, due to gravity) but may also be incorporated to some degree in the other axes to accommodate the intended circumstances of usage. For example, counterbalancing may be employed in the pitch joint of the wrist, and in vertically moving joints in the arm. The counterbalancing mechanisms may be comprised of any type of system known in the art such as a pneumatic system, simple springs, complex springs, counterweights, or systems of cables and springs with reels.

**FIGURE 16** is a block-diagram functionality-sketchwith four sub-figures 16A, 16B, 16C, and 16D--of the center-of-gravity garment attachment concept, where side views 16A, 16C and frontal views 16B, 16D are offered of an axle-based and ball-in-cup-based suspension system, in accordance with the present invention. Previous discussions have focused on the suspension of the shield/garment device from the upper portion of its frame. Now we will expand upon suspension mechanisms that are in close proximity to the center of gravity of the shield/garment device in order to improve its motion with regard to certain operator motions such as bending forward or sideways.

When a heavy object is suspended near its top (the point farthest from the earth and its gravitational pull), that object will not easily bend or tilt because such changes in orientation require forces in partial opposition to the gravitational forces. An operator may wish to partially bend over sometimes, as when leaning over the patient table a bit to reach for something. The operator would encounter the forces described, inhibiting the motion somewhat, although not preventing it. It is desired to limit these forces and render the shield/garment system easily tilted and/or bent (or creased or folded). This difficulty can be alleviated by suspension about an object's center of gravity or by a point near its center of gravity.

One way to address this is to attach the suspension mechanism to the shield/garment system at its center of gravity. Depending on the type of joint used, this can lead to great ease of tilting or bending in some or all directions because gravitational forces are cancelled out, and the only force required is the minimal force required to overcome friction and accelerate the object into motion. To prevent unwanted free rotation, friction at the joint may be incorporated to desired level, and/or the device can be suspended at a point slightly above the center of gravity so that it tends to orient itself vertically but still requires only minimal force to rotate it. In **FIGURES 16A-16D****,** a typical suspension assembly is shown for the support of a load, which includes an axle system shown in **FIGURES 16A** and **16B****,** and a pivot system shown in **FIGURES 16C** and 16D. Such a system may be a rigid structure such as a rod (shown) or a flexible structure such as a wire rope. Flexible wire rope would allow more freedom of rotation in the YZ plane, which can be desirable or undesirable depending on the situation of use.

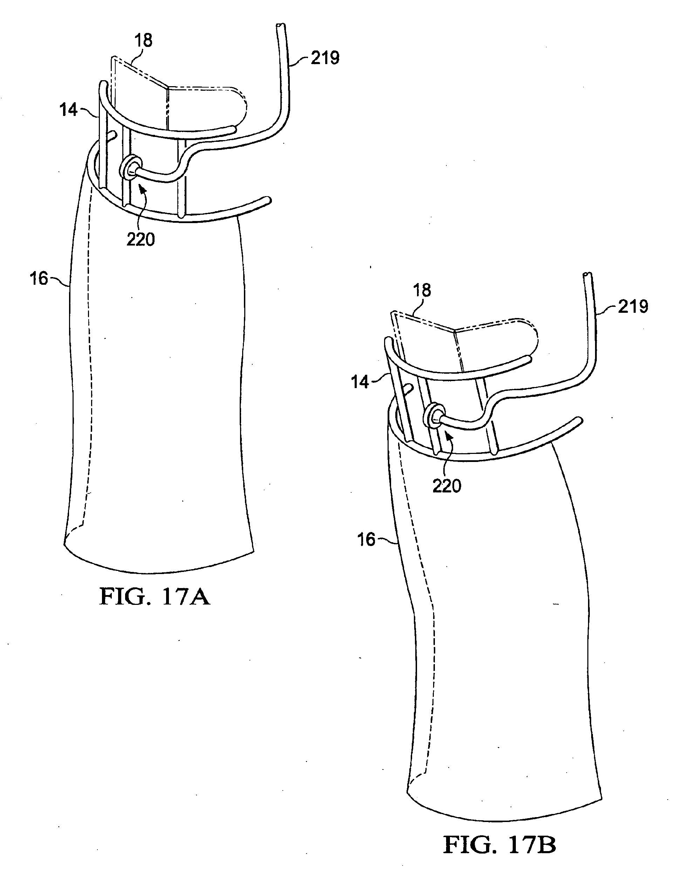

For example, **FIGURE 17** offers perspective views of a ball-in-cup center-of-gravity attachment of the frame 14 to a suspension arm 218, with the left perspective view **(****FIGURE 17A****)** showing the garment 16 in a natural and upright position, and the right perspective view **(****FIGURE 17B****)** showing the garment 16 in a sideways-bending stance, and where the suspending arm 218 retains the same basic orientation in both instances, in accordance with the present invention. In this embodiment, there is a ball-in-socket joint 220 connecting a rigid suspension arm 218 to the frame 14. The garment portion covering the frame 14 has been removed to depict the frame 14. The center of gravity is high due to the density of the frame and garment making the overall shield/garment/frame system top heavy. By utilizing the ball-in-cup joint 220, the operator can accomplish side-to-side bending and forward bending while the shield 18 and garment 16 remain in proper orientation relative to the operator, and little resistance to these motions is encountered.

The ball and cup joint 220 could be replaced by many different types of joints, such as needle in cup (with needle oriented with a vertical bias, and the cup in a corresponding manner), various manner of bearing or bushing joints, a universal type joint, an axle configuration, or a simple flexible connector such as a wire rope or strap which would permit forward bending. In the event that the center of gravity is located behind the front of the garment 16, possibly at a location that is extremely close to the operator's chest or torso, or perhaps within the operator's body, then it would not be feasible for an attachment at that exact location. However, several practical solutions are possible that permit iso-gravitational freedom of rotation in all planes. Likewise, a counterweight could be added to change the center of gravity forward to a more practical location for attachment. The counterweight would be integrated with the frame 14 in a manner to accomplish this goal, and could be placed as an extension of the frame 14 in any direction to increase moment arm of the counterweight, thus permitting a lighter counterweight to be used. The attachment could be as close as practical to the center of gravity. It does not need to be exactly at the center of gravity, because by being close to it, the forces required to tilt the device would still be minimal, especially since the overall weight of the device is expected to be less than 40 lbs.

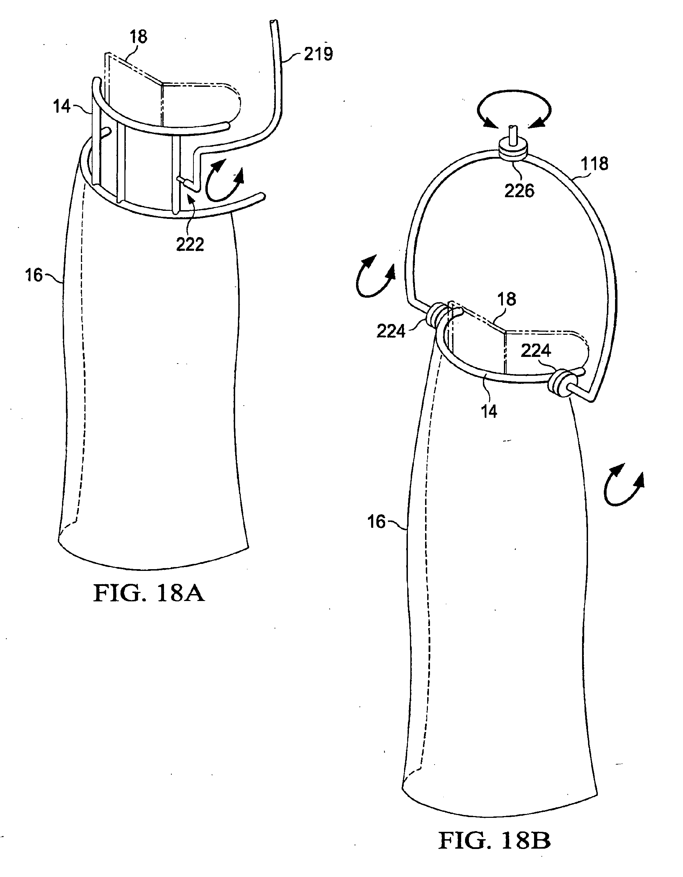

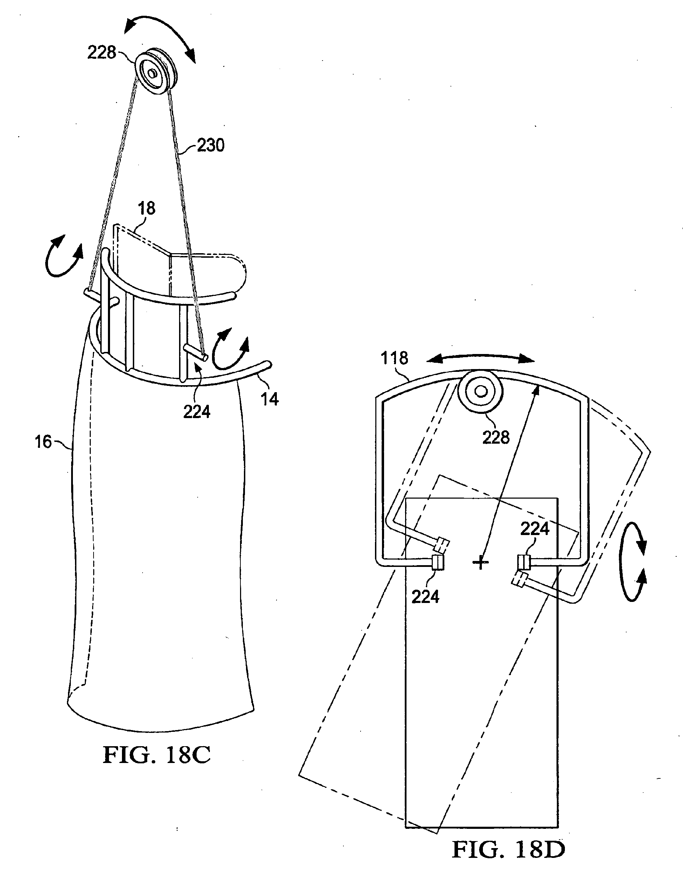

As another example embodiment, **FIGURE 18** offers perspective views of axle-based center-of-gravity attachment of the garment frame to a suspension component, with the left perspective view **(****FIGURE 18A****)** showing attachment via unilateral axle, the next perspective view **(****FIGURE 18B****)** showing attachment via bilateral axles and swiveling hanger, the next perspective view **(****FIGURE 18C****)** showing attachment via bilateral axles, and the right schematic view **(****FIGURE 18D****)** showing the wire ropes replaced with a rigid frame whose top portion is described by an arc with its center at the center of gravity, at the level of the pivot joints, in accordance with the present invention. An axle system could be employed to provide attachment location at the center of gravity in the Z axis (vertical). As seen in **FIGURES 18A** and **18B****,** this would therefore allow excellent forward bending function, without facilitating sideways bending, which may be less important or even undesirable in some situations. The axles could be located at the side(s) only, without running through the center where the operator's body may be located. Another option could be to place suspension components on both sides, such as wire ropes 230 **(****FIGURE 18C****),** or rigid frame **(****FIGURE 18D****),** which could be looped or passed over an overhead pulley to provide sideways bending function with great ease. The axle joint could be a simple shaft-in-bearing housing allowing only rotational motion of the shaft, like a bicycle wheel axle. This would allow a unilateral suspension arm configuration which could allow pitch while preventing roll as might be desired in this configuration to maintain proper orientation of the device. Instead of an axle, any type of rotating joint allowing rotation could be used.

On the left in **FIGURE 18A****,** the unilateral axle 222 attachments to frame 14 are at the height of the center of gravity, but it is suspended just left of it. This would allow easy forward bending for the operator, but sideways bending would not be markedly facilitated. In **FIGURE 18B****,** bilateral pivots 224 are present slightly above and to the left of right of the center gravity of a frame 14 with a different shape. This allows similar function as the embodiment shown in **FIGURE 18A****,** but with the option for using less bulky and strong unilateral support members. A pivot joint 226 rotating in the horizontal plane is integrated with the hanger 118, enabling yaw in the system allowing the operator to twist his body along with the system. In **FIGURE 18C****,** bilateral attachments 224 to frame 14 at or near the center of gravity may be rigid since they are connected to wire rope 230. They do not need to rotate as axles if the wire rope attachment allows rotation. The pulley system 228 overhead allows the system to bend sideways. Yaw would be enabled by flexibility in the wire rope 230, or the placement of a rotating joint above the pulley that allowed its rotation in the horizontal plane, similar as that depicted in **FIGURE 18****B.** This embodiment has somewhat similar function as the ball-and-cup joint at the precise center of gravity, without the need for placing a joint at or near the point of the center of gravity for the system, which may lie within the operator's body or otherwise inaccessible.

In another embodiment shown in **FIGURE 18D****,** wire rope is not used. Instead, a rigid hanger frame 118 may slide on a pulley 228. The hanger frame 118 includes an arc whose center is coincident with the center of gravity for the system, thus free rotation (roll) is allowed with very little force or change in weight bearing by the pulley 228. Not shown in this schematic are possibilities that facilitate its use, such as shaping of the lower hanger component to accommodate arm movement. The vertical component of the hanger may have many different shapes to facilitate fit and function, so long as the top arc is defined by its center at the center of gravity, and its highest point is directly over the center of gravity in the vertical Z axis. The pivot joints 224 allow pitch, the pulley 228 and arc hanger frame 118 allow roll, and yaw can be enabled using a pivot above the pulley as previously described, giving full ability to rotate about the center of gravity in all planes.

When using a harness and binding system as described previously, the binding components may benefit from being placed at the same height as the center of gravity attachment. This will facilitate the linkage between operator and device with regard to bodily motion in the XY plane, such as with walking forwards or sideways. If the binding components are not at the same level as the attachment at the center of gravity, such motions of the operator will exert forces on the device that are sufficiently large to cause rotation about the attachment site, creating undesirable motions since in this situation, the operator would want the device to remain in its neutral orientation while it followed the operator's body as they walked. This undesirable effect would be more important for ball-and-cup type joints, than for a purely axle- or pivot-type joint as in the **FIGURES 18A** and **18B****,** where sideways motion of the operator would not cause roll of the garment as it might with a ball-and-cup. Therefore, these designs could be useful when the operator binding site is not near the center of gravity. In other embodiments, the preferred attachment site may be slightly above the center of gravity of the system, so the device remains in its neutral orientation for most operator motions in the XY plane, but is still amenable to tilting when the operator bends forward or sideways. In addition to the embodiments shown in **FIGURES 18A-18D****,** a simple tether suspension could be used, such as a wire rope attached directly to the system at its center of gravity.

**FIGURE 19** is a perspective side view of a portable, floor-based, non-overhead suspension system 232 having a mobile floor stand 236 with an upright post 238 and manipulator arm 234 for distal attachment and suspension of a shield/garment 18,16, via frame 14, substantially about the garment's 16 center of gravity, in accordance with the present invention. For better stability, the mobile floor stand 236 includes weights 240 along a broad wheelbase 244. The wheelbase 244 has locking (or lockable) wheels 242 for securing the base 244 in position on the floor. Also, the wheelbase 244 is constructed of sufficient dimension and properly counterbalanced with sufficient weight to prevent tipping when the balancing arm 234 is fully extended and laden with the shield/garment 16.

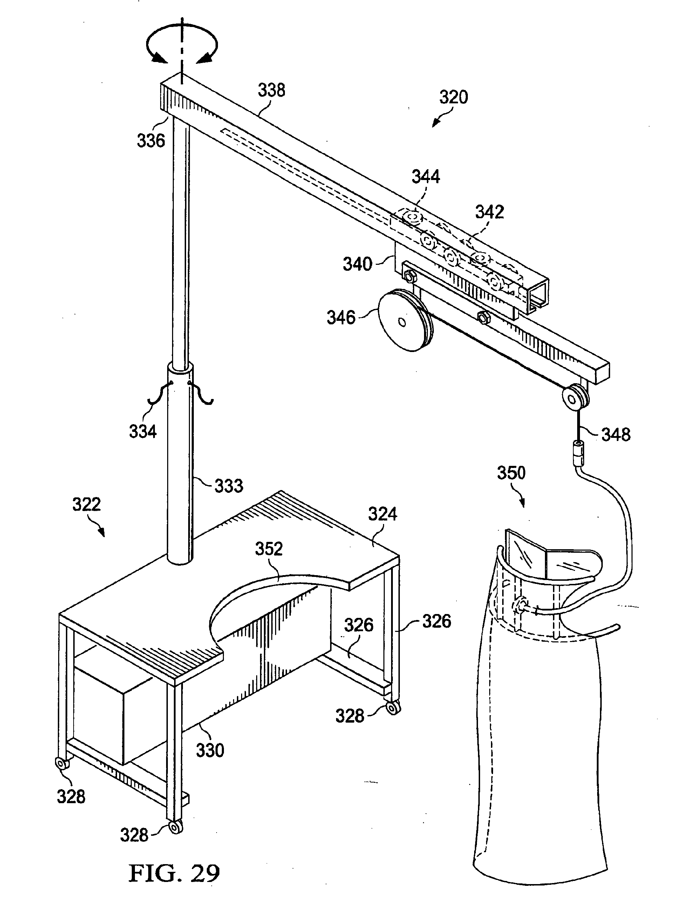

The balancing arm 234 is substantially horizontal and approaches the operator and shield/garment system from the side or back, rather than from overhead. In other embodiments, the arm could approach front the front. In this embodiment, the end effector 219 (end of the balancing arm) approaches the operator from the side at the level of the chest, then curves around the front, and attaches at the center of gravity of the frame of the shield/garment using a ball-and-cup joint 220. This system allows movement of the operator in the procedural area defined by an arc with a radius corresponding to the length of the arm. By putting the stand or mounting system a few feet from the operator, the operator's feet and body are not at risk of accidentally bumping into any part of the suspension system. This offers great advantages over any floor mounted system lacking the ability to distance (via articulating balance arms, for example) the operator wearing the shield/garment 18,16 from the bulk of the suspension system. A modified coat hanger, for example, might be capable of suspending a shield/garment so that the operator is not burdened by its weight. Such a modified coat hanger, however, would be in extremely close proximity to the operator. Without articulating balancing arms to distance the stand from the operator, the operator would be at great risk of inadvertently bumping into or tripping over parts of the stand. This system also offers advantages relative to an overhead floor based suspension system such as seen in **FIGURE 29****,** in that the post is not as tall and therefore need not be as wide to manage the same torque forces resulting from the arm and suspended system. Overall weight is reduced, and the system is more portable with fewer overhead collision issues with other apparatus. The system may also be transported through doorways without the need for telescoping posts.

While a ceiling-mounted system (as opposed to a floor-based system) would prevent any possibility of tripping over floor-based suspension components, the floor-based portable system 232 shown in **FIGURE 19** has advantages over a ceiling mounted system, especially in some specific environments: crowded ceilings are not a problem with this embodiment; the device can be wheeled into different procedure suites in the same institution; there could be some cost savings related to the absence of ceiling mounting, or the need for reinforcement of the ceiling and expensive analysis of structural support; the absence of overhead structures can reduce risk of collision with other structures such as hanging lights, hanging shields, or a moving image intensifier, which is often angled obliquely towards the operator and is located over their head.

Other configurations are possible, including a very short post, or no vertical post, with the arm attached to the wheelbase of the stand and extending upward to the attachment site with the radiation protection system. The arm could also be affixed rigidly to a site on the floor, wall, patient table, or stable back table. Also, the arm may attach to the frame with many different types of joints described elsewhere or located in other locations on the frame (e.g. an axle or pivot joint with one degree of freedom on the right side of the frame allowing forward tilt of the operator). The frame could be altered to allow a lower point of connection, by being extended more inferiorly towards the floor.

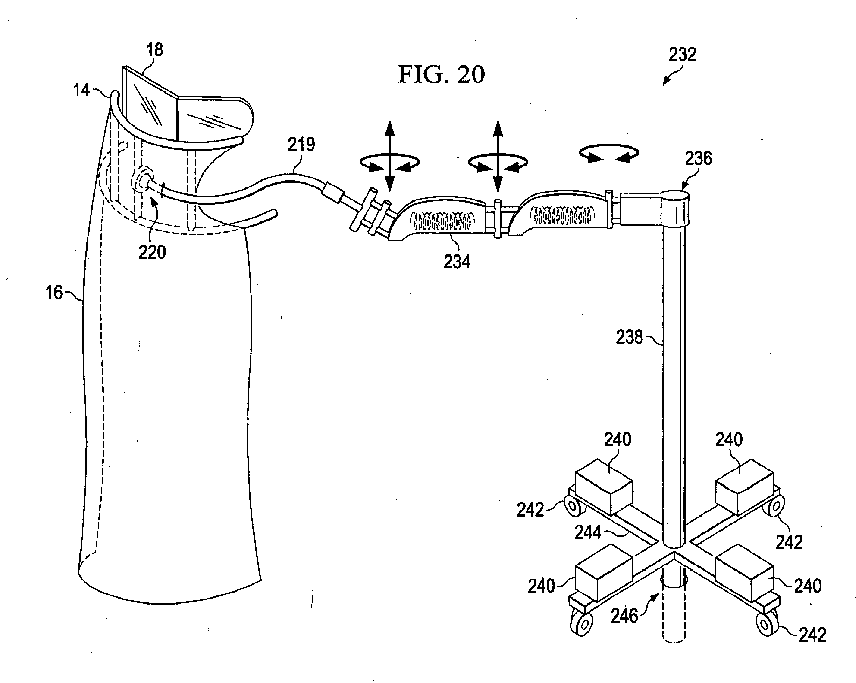

**FIGURE 20** is a perspective side view of the portable, floor-based, non-overhead suspension system of **Figure 19** modified for floor-based docking, in accordance with the present invention. By extending downward a floor-docking post or pole 246 from the bottom of the floor stand 236 into a receiving sleeve or channel in the floor or ground, the mobile floor stand 236 can be securely docked in place. The floor-docking post 246 increases the suspension system's ability to withstand torque applied to the end of the balancing arm 234 due to the weight of the suspended shield/garment/frame components 18,16,14. When not docked, the system 232 could be unstable with the arm 234 extended and load 18, 16, 14 attached. To permit undocking and portability without removal of the load 18, 16, 14, a system could be employed involving a locking mechanism for the portion of post 238 docked under the floor level 296, such that it cannot be telescoped back into the base 244 until unlocked. Unlocking would be enabled only when the arm 234 is swung in so that the supported weight is positioned very close to the post 238, thus reducing torque. Once the post 238, 246 is retracted and undocked, the arm 234 would lock so that it could not be extended to make the system 232 unstable. The arm 234 could be unlocked once the post 238, 246 is docked again. This safety mechanism can be incorporated into each of docking system embodiments described herein. Although **FIGURE 20** depicts one embodiment of this floor docking mechanism with a non-overhead suspension system, the floor supported system could be utilized with any of the described arm or jib systems, including overhead suspension systems. It could also be utilized with a bridge system where it might offer advantage if a floor-based bridge system were set up in a cantilevered fashion requiring stabilization as could be offered by this floor docking mechanism.

Other types of floor docking mechanisms are possible. Any number of the wheels could be designed to bind or attach securely to components on the floor to provide stability and prevent translational or rotational (tipping) motion of the stand. Other extensions or plurality of extensions from the device could attach as described above for the wheels. The attachments could be rails allowing some degree of motion in some directions. Any type of securable attachment system could be envisaged using mechanisms widely known in the art.

**FIGURE 21** is a perspective side view of the portable, floor-based, non-overhead suspension system of **FIGURE 19** modified for ceiling-based docking, in accordance with the present invention. By extending upward a ceiling-docking post or pole 252 from the top of the floor stand 236 into a receiving sleeve (e.g. a ceiling-post sleeve 250) or channel in the ceiling 248 or other ceiling-mounted structure, the mobile floor stand 236 can be securely docked in place. The ceiling-docking post 252 increases the suspension system's ability to withstand torque applied to the end of the balancing arm 234 due to the weight of the suspended shield/garment/frame 18, 16, 14. The diameter and bulk of the post 252 can be substantially reduced, and the wheelbase and weight of the base may also be reduced relative to non-docking systems. In the embodiment shown, the floor docking mechanism is depicted with a non-overhead suspension system; however, it could be utilized with any of the previously described arm or jib systems, including overhead suspension systems. It could also be utilized with a bridge system where it might offer advantage if a floor-based bridge system were set up in a cantilevered fashion requiring stabilization as could be offered by this floor docking mechanism. Also, floor docking and ceiling docking mechanisms could be present in the same device.

Other types of ceiling docking mechanisms are possible. Other extensions or plurality of extensions from the device could attach to sites secured to the ceiling. The attachments could be rails allowing some degree of motion in some directions. Any type of securable attachments system could be envisaged using mechanisms widely known in the art.

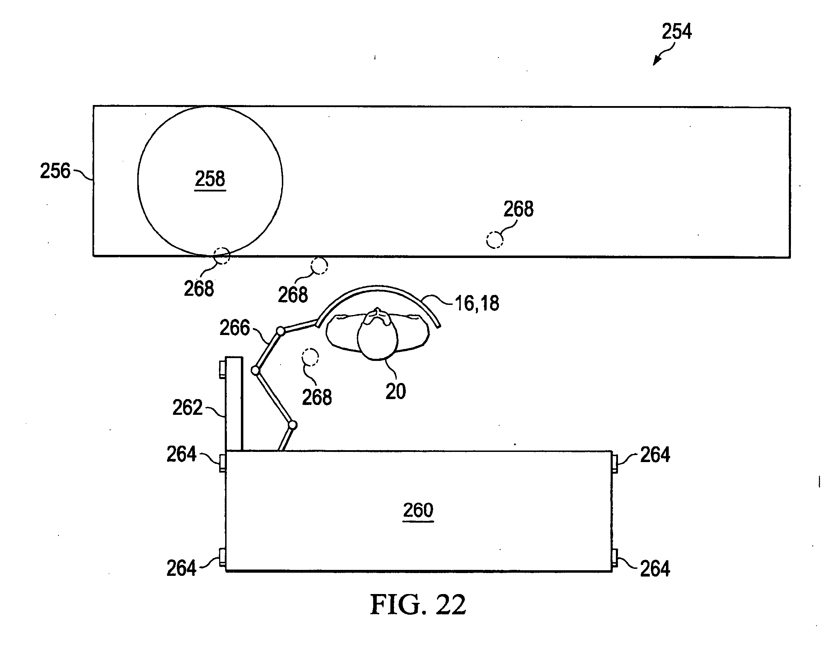

**FIGURE 22** is a perspective side view of a portable, floor-based, back-table-mounted garment suspension system with non-overhead suspension 254, in accordance with the present invention. The embodiment shown in **FIGURE 22** allows for articulated, balanced arm support of the operator's garment/shield 16,18 by relatively inexpensive modification of commonly used back tables. This helps reduce operator fatigue (due to the cumbersome weight of the shield/garment) without requiring the installation of a ceiling-based mounting system, such as a bridge crane and trolley, which can be quite expensive and/or complex, and without having to set aside precious floor space for a dedicated garment-holding floor stand. Such a back-table-mounted suspension system is portable, therefore it can be easily moved with the back table from one room to the next, or even simply moved out of the way when not in use.

A typical radiological examination room (and/or operating room) has a patient table 256 with an image intensifier 258 at one end of the room (which relative end will be referred to herein as the front of the room). The operator 20 generally works behind or adjacent the patient table 256 while wearing the radiation-blocking shield/garment 18, 16 with his or her back facing the back of the room. A back table 260 is located behind the operator 20 towards the back of the room and is used by the operator 20 to keep his or her tools and/or other equipment within convenient reach. With a few modifications to the typical back table--such as providing extended table legs (or outriggers) 262 for widening the table's base and providing locking wheels 264 and/or floor hooks--the back table 260 can be made stable enough to serve as a secure support platform for a shield/garment-suspending articulating arm and/or balancing arm 266 system. With a few modifications, it is also possible, in the alternative, to mount such a shield/garment-suspending articulating arm and/or balancing arm system at many different possible locations including on the patient table or even to a point on the floor as depicted. Although the depicted embodiment is shown with a non-overhead articulating arm, any type of arm or jib system, including overhead or non-overhead, could be modified and used in alternative embodiments.

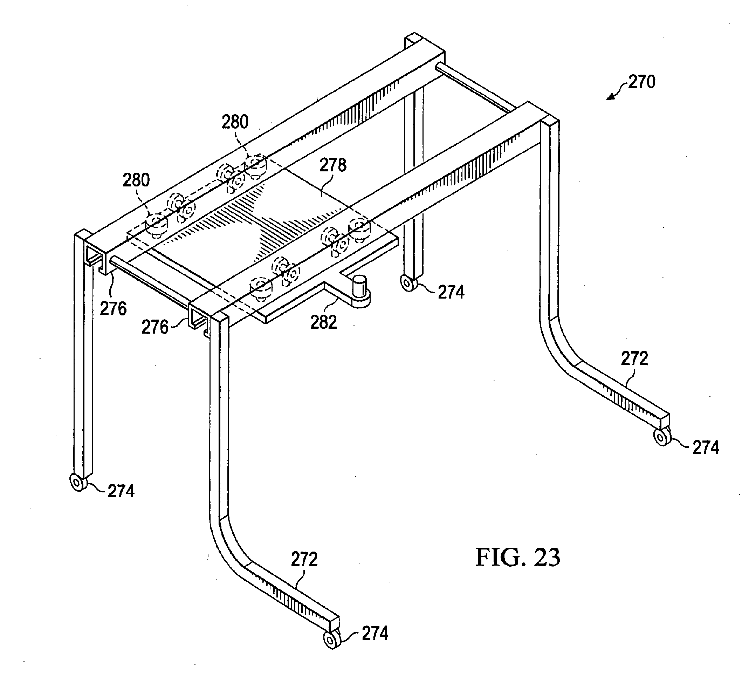

**FIGURE 23** is a perspective view of one embodiment of a portable track stand 270 for use with the back-table-mounted garment suspension system 254 shown in **Figure 22****,** where stability is provided by the track stand's broad base 272 and locking wheels 274, in accordance with the present invention. Stand 270 rolls multi-directionally on wheels 274. It contains two track rails 276 that allow a trolley 278 to move longitudinally along the track rails 276 via trolley wheels 280, while also providing the ability to withstand the moment arm forces produced by the weight of the balancing arm (mounted onto the trolley 278) and shield/garment device (not shown)at attachment point 282. Trolley wheels 280 may be lockable, so that the trolley can be locked in the desired working position so that further motion during work is only available in the arm system connected to stand 270. The top of the portable track stand 270 may be covered or otherwise modified to simultaneously serve as a back-table.

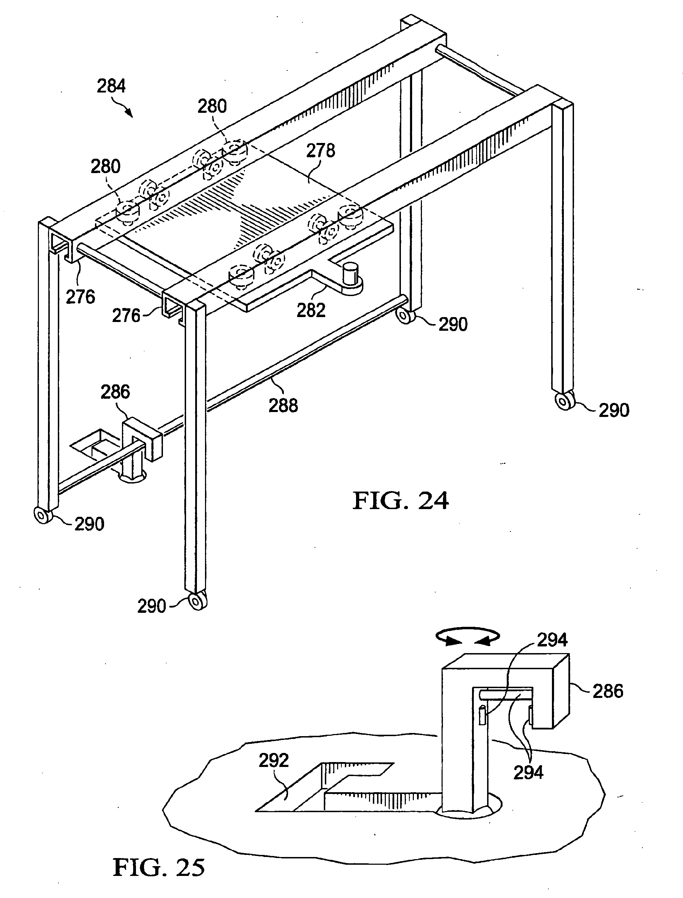

**FIGURE 24** is a perspective view of another embodiment of a portable track stand 284 (in the depicted embodiment, a floor-locking portable track stand) for use with the back-table-mounted garment suspension system shown in **FIGURE 22****,** where stability is provided by at least one stowable, recessed floor hook 286, in accordance with the present invention. In this embodiment of the portable track stand/table 284, there is attached to the floor a mechanism, hook 286, that can attach to and detach from a crossbar 288 along the base of the table 284 using simple operator movements. It stabilizes the table 284 by preventing the backside from elevating due to the torque created by the attached balancing arm and the suspended radiation protection device (not shown) at attachment point 282. This allows a substantial reduction in weight and/or wheelbase, of table for stability purposes and serves to further minimize unwanted lateral motion of the table 284 along the floor that might occur in small degrees even with the wheels 290 locked in place. One or more floor hooks 286 may be used in any of the portable stands or back table embodiments described herein and as required by the operational requirements. Alternative embodiments may provide a locking safety mechanism similar to that described in **FIGURES 20** and **21** which only permits unlocking from the floor when a load is stowed over or close to table, and the support arm cannot be extended unless locked to floor.

**FIGURE 25** is a perspective view of one variation of the stowable, recessed floor hook(s) 286, in accordance with the present invention. To prevent obstruction or tripping of personnel when the floor hook 286 is not being used, the floor hook 286 can fold down into a recess or inset 292 in the floor. To stow away the floor hook 286, the operator might, for example, first rotate the floor hook 286 ninety degrees (90°) counter-clockwise, then fold it down into floor. Once in the floor (i.e. within the correspondingly-shaped floor recess 292), the floor hook 286 would lie flat/flush with the floor without risk of being obstructive. In yet another variation of the floor hook 286, the hook 286 can freely rotate about its floor attachment, allowing the table 284 to change orientation while securely but rotatably held by the floor hook 286 to the floor. The floor hook 286 itself, along its hooking surfaces, can also have top rollers and side rollers ("top" and "side" relative to the table crossbar being hooked) 294 to allow the table 284, while hooked, to shift laterally--from side to side--along the floor (i.e. along its long axis) for increased range.

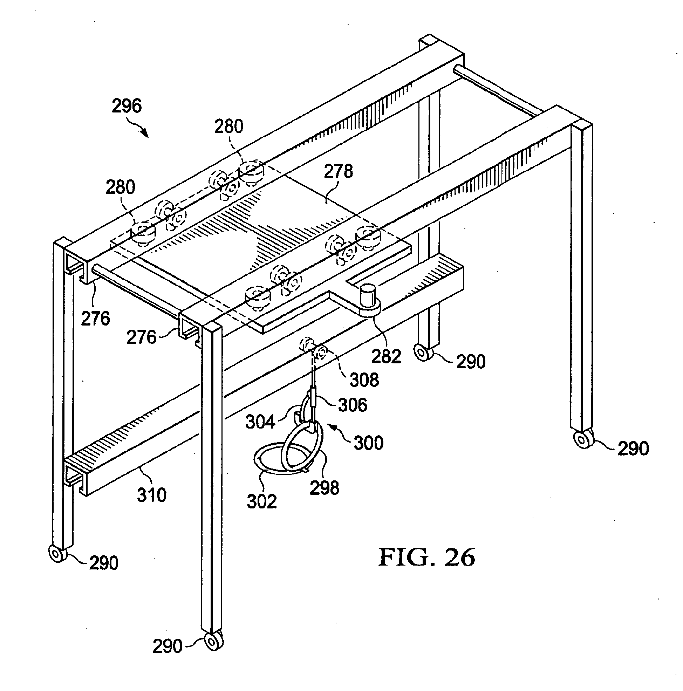

**FIGURE 26** is a perspective view of another embodiment of a portable track stand 296 (more specifically, a cam-locked portable track stand) for use with the back-table-mounted suspension system 254 shown in **FIGURE 22****,** where stability is provided by at least one stowable, recessed floor ring 298 and at least one cam-locking hook (or other cam-locking ring catch) 300, in accordance with the present invention. The floor ring 298 folds up from the floor inset (or recess) 302, and the cam-locking hook 300, which has an operating handle 304 for locking the hook 300 and tightening its hold, attaches to it. The operator squeezes the cam lock handle 304 closed. This action shortens and tightens the apparatus, pulling downward as it locks, to provide sufficient downward pull on the table 296 to prevent it from wobbling or lifting slightly off its wheels when weight is applied on the balancing arm. There is also a length-adjustor mechanism 306 on the shaft of the cam lock 300 to provide coarse adjustment of the length. This mechanism can be supported from a trolley 306 with trolley wheels 308 that can roll freely along the track 310 attached to the legs of the table 296. Such a rail system can be applied to any of the floor-docking table systems described herein. As in previous embodiments, this stand 296 can simultaneously serve as a back-table for procedural supplies by placing a tabletop on it, for example, and covering it with a sterile drape as is customarily done. A joint allowing rotation of the floor ring 298, and/or of the shaft for the cam-lock hook 300 that grips the ring 298, will allow rotation of the table 296 in the plane of the floor with the floor ring 298 as the center of rotation. There can be a safety mechanism to prevent unclamping of cam lock 300 while the shield/garment (or other load) is positioned on the balancing arm (not shown), as a sufficient torque applied against the balancing arm might undesirably allow the table 296 to tip. To prevent the need to remove the load, the balancing arm and its load could be placed into a parked position where the load is positioned substantially over the center of the table 296.

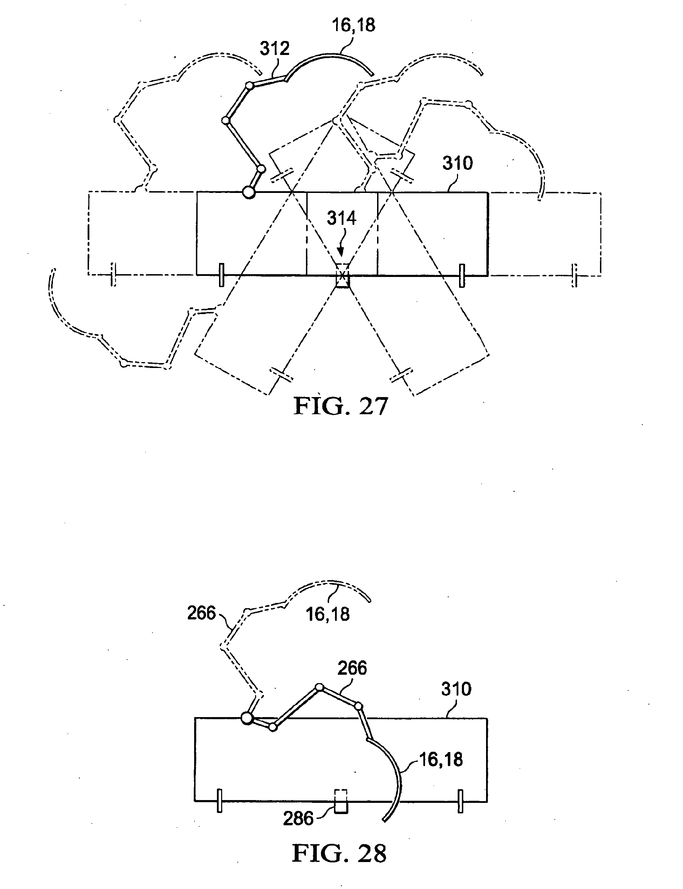

**FIGURE 27** is an elevated top view of the range of motion available to the portable track stand 310 for use with the back-table-mounted garment suspension system 254 shown in **FIGURE 22** and with the floor-securing means depicted in **FIGURES 24-26****,** in accordance with the present invention. The floor hook 314 is positioned near the middle of the table 310 in the neutral position, hooking the crossbar of the table as previously described. The table can be rolled to the left or right, or rotated around the hook 314 as shown, giving great range to the balancing arm 312 and garment/shield 16,18 attached thereto.

**FIGURE 28** is a perspective view of the portable, floor-based, back-table-mounted garment suspension system 254 shown in **Figure 22** where an example table-mounted manipulator arm 266 is depicted in two positions: an operating position and a parked position, in accordance with the present invention. The table 260 may be unhooked from the floor when tension is released from the hook mechanism 286, indicating the table 260 is stable. This can be achieved by removing the load, or in this instance, by rotating the load substantially over the table 260 to remove moment arm or torque forces. Another embodiment of safety lock could involve a mechanical linkage between a table-to-floor locking mechanism, and the arm such that the locking mechanism can unlock only when the arm is swung into a safe position where the table is stable without tethering to the floor. The device can swing over or under the table 260. The latter has the advantages of space savings, lower center of gravity, non-obstruction of the tabletop area, and better aesthetics. The garment 16 may be constructed to roll or fold up so that its vertical distance is reduced, allowing the device to park more easily in the space available.

**FIGURE 29** is a perspective view of a back table 322 combined with a jib arm and trolley suspension assembly for supporting the radiation protection system. In this embodiment, a frame 326 on wheels 328 supports a column 333 for a jib-boom system. A table top 324 is connected to the frame 326 and serves as the back table 322 for the operator's supplies. Underneath the table top 324 is a compartment 330 containing weights and a motor system to move the system 320 via powered wheels 328. A sterile drape may be placed over the table top 324 as is common in the art. The column 333 may also include hooks 334 for hanging medical supplies or instruments. The column 333 may telescope to change its height, and which may be facilitated with a counter-balance system 346 (of any type, but the use of counterweights may be advantageous to provide more weight and stability) to negate the weight of the upper column 333, boom 338, and suspended components 350 (e.g., garment/shield). A rotating joint 336 between the boom 338 and the column 333 allows rotation of the boom 338 in the horizontal plane. In this embodiment, the boom 338 is telescoping with an integrated trolley 340 with anti-kickup wheels 342 and side rollers 344, although a conventional boom could be used. A trolley 340 has linear motion along the boom 338. The radiation protection system 350 is suspended by a wire (or line) 348, and its weight is counterbalanced by a balancer 346 attached to the trolley 340, and placed rearward for counterbalance advantage in this embodiment. The entire system 320 allows free motion of the protection system 350 in the X, Y, and Z space. The system 320 may be moved into any position in the workplace by the motorized drive and at least two of the four wheels 328 are provided so as to allow steering. The table 322 is deep to create a long wheelbase in the short axis, to increase stability. In this embodiment, the table's front has an arced cut-out 352 to permit more ergonomic usage by the operator despite its deep shape.

System 320 may prove advantageous over a separate floor mounted jib boom and back table because much space is saved, and it may be possible to put the post at any location by design, rather than working around the table. This may allow a shorter boom to reach the area of use, allowing less force to move it, and less bulky construction of the boom and base. Likewise, it may be possible to situate the support closer than a ceiling mounted system where ceiling obstacles may necessitate longer booms. It also obviates the need for ceiling tracks since the post may be moved by moving the table. Many variations of this system are possible including different shapes of the frame or table, numbers of floor contacts or wheels, function of column including any of the various mechanisms described elsewhere such as ceiling or floor docking, conventional boom with conventional trolley and balancer arrangement directly below trolley, absence of motorized drive, use of any other type of arm mechanism including robot arms, holonomic arms, or articulating arm systems of all types, or arms extending horizontally from the table/stand and attaching in a non-overhead manner as described in FIGURE 20, or any of numerous mechanisms of counterbalancing the radiation protection system.

The telescoping boom has the advantage of permitting the end of the boom to be retracted when overhead obstacles are encountered, such as the image intensifier, as seen in the **Figure 22****.** With the telescoping boom design in **FIGURE 29****,** the operator is directly underneath the end of the boom, so the boom end will not collide with objects that are not directly overhead of the operator. There are many possible modifications and variations of the mobile floor stand system and/or the manipulator-type arm discussed thus far.

If desired another stabilization and safety mechanism can be built-in to the portable floor-based suspension system: the base, stand, or table can attach to the floor via a component that slips down into a mechanism in the floor that grips it. This could be a hole in the floor with internal teeth or some other mechanical binding system to grip a table component--such as a rod or key--that inserts into it. A plurality of these mechanisms could be employed. The action of locking the table to the floor can be the required mechanism for unlocking another lock on the tabletop, which allows the articulating/manipulator/balancing arm to swing out from its docked position over the table. The arm would then be available for suspending the radiation shield/garment for operations. After use, the shield/garment/apron-suspending arm could then be swung back over the table top so that the table, arm, and garment are stable without the floor lock. When docked over the table, a lock can automatically activate to keep all components in position. If desired, the docking and locking action can also simultaneously deactivate the locking/binding mechanism in the floor so that it is unlocked, thus releasing the table from the floor and allowing it to be moved.

As discussed above, **FIGURES 19-29** depict a variety of suspended radiation protection devices wherein a significant portion of the weight of the apparatus is supported by the floor, although it is contemplated that many alternative embodiments are possible within the scope of this invention. It is noted that alternative embodiments may include various components disclosed herein. For example, any of the different overhead or non-overhead suspension systems disclosed herein may be used on any device. Jib arms may be used where articulating arms are described, and vice versa. Motorized components may facilitate movement of the tables or stands, or movement of the suspension components such as the trolley. Other devices commonly known in the art besides wheels may be used to facilitate table motion, such as belts. A track may be present in the floor that supports and guides the table, which may ride on the tracks on wheels, rollers, or other common mechanisms. The tables or stands may be stabilized by secure, detachable attachment of one or more legs or components to the floor in fixed locations, wherein the tables or stands may not be slid or rolled about the floor once attached. This could also stabilize the table from tipping due to the cantilevered load.

In various contemplated embodiments, stability may be provided by detachable or non-detachable connections to the tracks upon which the devices ride, slide or roll. Wheels, rollers or bearings positioned on the legs, or the bottoms of the stands or tables, may be capable of sliding or rolling inside a track which is attached to the floor, or imbedded within the floor. This track could be securably attached to the floor so that the table or stand, once engaged in the track, could not be pulled away from the floor without substantive force, and therefore would not tip or fall. This engagement could be permanent, or semi-permanent and accomplished using mechanisms widely known in the art. A safety mechanism such as a mechanical lock or detent could be employed to prevent accidental dislodgement and de-stabilization of the device. Likewise, a single track or multiple tracks could be used for stabilizing the system disclosed herein. In the event of a single track, it could be attachable to the legs or side of the table opposite the suspended load, to prevent that side from rising up.

Stabilization could also be provided through different means. A counterweight may be present in a remote location from the remainder of stand, base, table, or suspended apparatus. For example, it could be attached to a rigid arm extending in the opposite direction relative to the suspended device, thus counterbalancing it. It could be positioned above head level to remain free of personnel motion. It could be positioned lower to be free of obstructions higher in the room. The combination of weight and arm length could be chosen to provide the necessary counterbalancing effect while addressing other logistical considerations of each application or operating suite. The counterweight arm could be stationary, or it could be movable to allow optimization of its position. It could be linked with the arm for the suspended radiation protection device to remain in ideal counterbalancing position during use of the system.

While the invention has been particularly shown and described with reference to a various embodiments, it will be understood by those skilled in the art that various changes in form and detail may be made therein without departing from the scope of the invention, as defined in the appended claims.

## Claims

1. A protection apparatus, comprising:
a protection system (10) having:
a garment (16) that substantially contours to an operator's body wherein the garment (16) is operable to protect the operator (20) from a substantial portion of radiation;
a curved frame (14) from which the garment is suspended; and
a suspension assembly (12) operable to support the protection system (10);
**characterised in that**
the protection system (10)further comprises a first binding component (36; 38; 44; 60) of a binding assembly operably attached on or about the protection system (10),
the protection apparatus further comprises a second binding component (34; 52) of the binding assembly operably attached on or about the operator (20) operable for engaging the operator (20) and the protection system (10),
and **in that** the frame (14) is adapted to contour to the operator's body.

2. The apparatus of claim 1 wherein the second binding component (34; 52) comprises a harness (32) which may comprise a belt, vest, strap, garment or other structure capable of being detachably secured on or about the operator (20).

3. The apparatus of claim 1 or claim 2 wherein the curved frame (14) is constructed from rigid, jointed semi-rigid and/or flexible material to allow the frame (14) to substantially contour to the operator's body.

4. The apparatus of claim 1 wherein the suspension assembly is one selected from the group consisting of:
a) a trolley (72);
b) an articulating arm;
c) a balancer (80);
d) a reaction arm;
e) an extension arm (92);
f) a jib crane;
g) a wire rope (82);
h) a bridge crane; and,
i) a manipulator arm (190).

5. The apparatus of claim 1, further comprising:
a binding assembly wherein the binding assembly is one selected from the group consisting of:
a) a ball and cup connection
b) a guide block (56)
c) a guide finger (64)
d) a magnet (66)
e) a strike plate (58)
f) a friction fastener
g) a magnetic fastener
h) a hook and loop fastener and
i) an electromagnetic fastener;

6. The apparatus of claim 1 further comprising a locking mechanism operable to substantially immobilize at least one component of the suspension assembly.

7. The apparatus of claim 1 wherein a face shield (18) is movably connected to the frame (14).

8. The apparatus of claim 1 further comprising a mobile floor stand (236) integrated with the suspension assembly.

9. The apparatus of claim 1 wherein the frame (14) is substantially flexible.

10. A method, comprising:
suspending with a suspension assembly a radiation protection system which includes a garment (16) secured to a frame operable to protect an operator from a substantial portion of radiation;
engaging the operator (20) with the protection system with a binding assembly
**characterised in that**
the binding assembly includes a first binding component (36; 38; 44; 60) attached on or about the protection system and a second binding component (34; 52) attached on or about the operator (20) and
a portion of the frame (14) and the garment (16) is contoured to the operator's body.

11. The method of claim 10 wherein the suspension assembly is one selected from the group consisting of:
a) a trolley (72);
b) an articulating arm;
c) a balancer (80)
d) a reaction arm;
e) an extension arm (92);
f) a jib crane;
g) a wire rope (82); and,
h) a bridge crane and
i) a manipulator arm.

12. The method of claim 10 wherein the operator (20) engages with the protection system by moving the first (36; 38; 44; 60) and second (34; 52) binding components into proximity with each other such that the binding assembly detachably secures the operator to the protection system and the operator disengages from the protection system by pushing away from the frame (14) resulting in the detachment of the first and second binding components.

13. The method of claim 10 wherein the operator (20) engages and disengages with the protection system by activating or deactivating an
electromagnetic binding assembly.

14. The method of claim 10 wherein the binding assembly is a selected one from the group consisting of:
a) hook and loop fasteners;
b) friction fasteners;
c) mechanical fasteners;
d) magnetic fasteners;,
e) electromagnetic fasteners,
f) a ball and cup connection
g) a guide block (56)
h) a guide finger (64)
i) a magnet (66) and
j) a strike plate (58).

15. The method of claim 10 wherein the suspension assembly is detachably connected to a mobile floor stand.

## Patentansprüche

1. Ein Schutzapparat, bestehend aus:
Einem Schutzsystem (10) mit:
Einer Bekleidung (16) die sich substanziell dem Körper des Betreibers profiliert/anpasst, wobei die Bekleidung (16) funktionsbereit ist, um den Betreiber (20) vor einer substanziellen Menge an Strahlung zu schützen;
din gebogener Rahmen (14) von dem die Bekleidung hängt; und eine Hängearmaturenhalterung (12) die funktionsbereit ist, um das Schutzsystem (10) zu unterstützen, welches davon **dadurch gekennzeichnet, dass**
das Schutzsystem (10) desweiteren aus einem ersten Bindeelement (36; 38; 44; 60) einer Bindungseinheit, die funktionsbereit an oder über dem Schutzystem (10) angebracht ist, besteht,
der Schutzapparat besteht desweiteren aus einem zweiten Bindeelement (34; 52) der Bindungseinheit, die funktionsbereit ist und an oder über dem Betreiber befestigt ist und zur Aktivierung des Betreibers (20) funktionsbereit ist und dem Schutzsystem (10),
und darin, dass der Rahmen (14) sich an den Körper des Betreibers anpasst.

2. Der Apparat des Anspruchs 1, worin das zweite Bindeelement (34; 52) aus einem Geschirr (32) besteht, das aus einem Gurt, einer Veste, Riemen, Gewand oder anderer Struktur besteht, die in der Lage ist abnehmbar an oder über den Betreiber (20) gesichert zu sein.

3. Der Apparat des Anspruchs 1 oder Anspruchs 2, worin der gebogene Rahmen (14) aus einem starren, gegliederten halb-starren und/oder flexiblen Material hergestellt ist, was dem Rahmen (14) ermöglicht, sich substanziell der Kontur des Körper des Betreibers anzupassen.

4. Der Apparat des Anspruchs 1, worin die Hängearmaturenhalterung aus einer Gruppe ausgewählt ist aus:
a) Einer Laufkatze (72);
b) Einem Gelenkarm;
c) Einem Stabilisator (80);
d) Eine Drehmomentstütze;
e) Einem Verlängerungsarm (92);
f) Einem Auslegerkran;
g) Einem Drahtseil (82);
h) Einem Brückenkran; und
i) Einem Manipulatorarm (190).

5. Der Apparat des Anspruchs 1, besteht desweiteren aus:
Einem Bindeelement worin das Bindeelement aus einer Gruppe ausgewählt wurde bestehend aus:
a) Einem Ball- und Becheranschluss
b) Einem Führungsschlitten (56)
c) Einem Führungsfinger (64)
d) Einem Magnet (66)
e) Einem Schließblech (58)
f) Ein Reibungsverschluss
g) Ein Magnetverschluss
h) Ein Klettverschluss und
i) Einem elektromagnetischen Verschluss;

6. Der Apparat des Anspruchs 1 besteht desweiteren aus einer Arretierung (Verriegelung), die funktionsbereit ist um substanziell mindestens eine der Elemente der Hängearmaturenhalterung zu immobilisieren.

7. Der Apparat des Anspruchs 1, worin ein Gesichtsschutz (18) beweglich mit dem Rahmen (14) verbunden ist.

8. Der Apparat des Anspruchs 1 besteht desweiteren aus einem mobilen Bodenstativ (236), das mit der Hängearmaturenhalterung integriert ist.

9. Der Apparat des Anspruchs 1, worin der Rahmen (14) substanziell flexibel ist.

10. Eine Methode bestehend aus:
Aussetzen mittels einer Hängearmaturenhalterung ein Strahlungsschutzsystem, das ein Gewand (16) beinhaltet, das an den Rahmen gesichert ist, um funktionsbereit den Betreiber vor einer substanziellen Menge an Strahlung zu schützen;
Aktivierung des Betreibers (20) mit dem Schutzsystem mit einem Bindeelement, das **dadurch gekennzeichnet, dass**
das Bindeelement schließt ein erstes Bindeelement ein (36; 38; 44; 60), welches an oder über dem Schutzsystem angebracht ist und einem zweiten Bindeelement (34; 52), welches an oder über dem Betreiber (20) angebracht ist und
ein Abschnitt des Rahmens (14) und dem Gewand (16) ist der Kontur des Körpers des Betreibers angepasst.

11. Die Methode des Anspruchs 10, worin die Hängearmaturenhalterung aus einer Gruppe ausgewäht ist, bestehend aus:
a) Einer Laufkatze (72);
b) Einem Gelenkarm;
c) Einem Stabilisator (80);
d) Eine Drehmomentstütze;
e) Einem Verlängerungsarm (92);
f) Einem Auslegerkran;
g) Einem Drahtseil (82); und
h) Einem Brückenkran und
i) Einem Manipulatorarm.

12. Die Methode des Anspruchs 10, worin der Betreiber (20) mit dem Schutzsystem interagiert, indem er die ersten (36; 38; 44; 60) und zweiten (34; 52) Bindeelemente aneinander eng annähert, so dass die Bindeeinheit den Betreiber abnehmbar an das Schutzsystem sichert und der Betreiber löst sich vom Schutzsystem ab, indem er sich vom Rahmen wegschubst (14), was in einer Ablösung der ersten und zweiten Bindeelemente resultiert.

13. Die Methode des Anspruchs 10, worin der Betreiber (20) mit dem Schutzsystem in Verbindung tritt oder sich von diesem loslöst, indem der eine elektromagnetische Bindeeinheit aktiviert oder deaktiviert.

14. Die Methode des Anspruchs 10, worin die Bindeeinheit aus einer Gruppe ausgewählt ist aus:
a) Klettverschlüsse;
b) Reibungsverschlüsse'
c) Mechanische Verschlüsse;
d) Magnetische Verschlüsse;,
e) Elektromagnetische Verschlüsse,
f) Einem Ball- und Becheranschluss
g) Einem Führungsschlitten (56)
h) Einem Führungsfinger (64)
i) Einem Magnet (66) und
j) Einem Schließblech (58).

15. Die Methode des Anspruchs 10, worin die Hängearmaturenhalterung abnehmbar mit einem mobilen Bodenstativ verbunden ist.

## Revendications

1. Un appareil de protection comprenant :
un système de protection (10) possédant :
un vêtement (16) qui sensiblement enveloppe le corps d'un opérateur où le vêtement (16) est conçu de façon à protéger l'opérateur (20) d'une partie substantielle d'un rayonnement,
un châssis incurvé (14) sur lequel le vêtement est suspendu, et
un ensemble de suspension (12) conçu de façon à soutenir le système de protection (10),
**caractérisé en ce que**
le système de protection (10) comprend en outre un premier composant de liaison (36, 38, 44, 60) d'un ensemble de liaison rattaché de manière opérationnelle à ou autour du système de protection (10),
l'appareil de protection comprend en outre un deuxième composant de liaison (34, 52) de l'ensemble de liaison rattaché de manière opérationnelle à ou autour de l'opérateur (20) conçu de façon à entrer en prise avec l'opérateur (20) et le système de protection (10),
et **en ce que** le châssis (14) est adapté de façon à envelopper le corps de l'opérateur.

2. L'appareil selon la Revendication 1 où le deuxième composant de liaison (34, 52) comprend un harnais (32) qui peut comprendre une ceinture, un gilet, une courroie, un vêtement ou une autre structure capable d'être fixé de manière amovible sur ou autour de l'opérateur (20).

3. L'appareil selon la Revendication 1 ou 2 où le châssis incurvé (14) est construit à partir d'un matériau rigide, semi-rigide articulé et/ou flexible destiné à permettre au châssis (14) d'envelopper sensiblement le corps de l'opérateur.

4. L'appareil selon la Revendication 1 où l'ensemble de suspension est un ensemble sélectionné dans le groupe se composant de :
a) un chariot (72),
b) un bras d'articulation,
c) un balancier (80),
d) un bras de réaction,
e) un bras d'extension (92),
f) une grue à flèche,
g) un câble métallique (82),
h) un pont roulant, et,
i) un bras manipulateur (190).

5. L'appareil selon la Revendication 1, comprenant en outre :
un ensemble de liaison où l'ensemble de liaison est un élément sélectionné dans le groupe se composant de :
a) une connexion à coupelle et à bille
b) un bloc de guidage (56)
c) un doigt de guidage (64)
d) un aimant (66)
e) une plaque antichoc (58)
f) une fermeture à friction
g) une fermeture magnétique
h) une fermeture à boucles et crochets, et
i) une fermeture électromagnétique.

6. L'appareil selon la Revendication 1 comprenant en outre un mécanisme de verrouillage conçu de façon à sensiblement immobiliser au moins un composant de l'ensemble de suspension.

7. L'appareil selon la Revendication 1 où un masque facial (18) est raccordé de manière déplaçable au châssis (14).

8. L'appareil selon la Revendication 1 comprenant en outre un socle de plancher mobile (236) intégré à l'ensemble de suspension.

9. L'appareil selon la Revendication 1 où le châssis (14) est sensiblement flexible.

10. Un procédé, comprenant :
la suspension avec un ensemble de suspension d'un système de protection contre le rayonnement qui comprend un vêtement (16) fixé à un châssis conçu de façon à protéger un opérateur d'une partie substantielle d'un rayonnement,
la mise en prise de l'opérateur (20) avec le système de protection avec un ensemble de liaison
**caractérisé en ce que**
l'ensemble de liaison comprend un premier composant de liaison (36, 38, 44, 60) rattaché à ou autour du système de protection et un deuxième composant de liaison (34, 52) rattaché à ou autour de l'opérateur (20), et
une partie du châssis (14) et du vêtement (16) enveloppent le corps de l'opérateur.

11. Le procédé selon la Revendication 10 où l'ensemble de suspension est un élément sélectionné dans le groupe se composant de :
a) un chariot (72),
b) un bras d'articulation,
c) un balancier (80)
d) un bras de réaction,
e) un bras d'extension (92),
f) une grue à flèche,
g) un câble métallique (82), et,
h) un pont roulant, et
i) un bras manipulateur.

12. Le procédé selon la Revendication 10 où l'opérateur (20) entre en prise avec le système de protection par le déplacement des premier (36, 38, 44, 60) et deuxième (34, 52) composants de liaison à proximité l'un de l'autre de sorte que l'ensemble de liaison fixe de manière amovible l'opérateur au système de protection et l'opérateur se libère du système de protection en se repoussant du châssis (14), ce qui résulte en le détachement des premier et deuxième composants de liaison.

13. Le procédé selon la Revendication 10 où l'opérateur (20) entre en prise avec et se libère du système de protection par l'activation ou la désactivation d'un ensemble de liaison électromagnétique.

14. Le procédé selon la Revendication 10 où l'ensemble de liaison est un élément sélectionné dans le groupe se composant de :
a) fermetures à boucles et crochets,
b) fermetures à friction,
c) fermetures mécaniques,
d) fermetures magnétiques,
e) fermetures électromagnétiques,
f) une connexion à coupelle et à bille
g) un bloc de guidage (56)
h) un doigt de guidage (64)
i) un aimant (66), et
j) une plaque antichoc (58).

15. Le procédé selon la Revendication 10 où l'ensemble de suspension est raccordé de manière amovible à un socle de plancher mobile.
